# EUROPEAN PATENT APPLICATION

(11) **EP 4 516 316 A1**
(43) Date of publication of application: **05.03.2025**
(21) Application number: 23796325.1
(22) Date of filing: 24.04.2023
(51) Int. Cl.: A61K 39/395, A61K 45/00, A61K 47/68, A61K 49/00, A61P 21/00, A61P 25/00, C07K 16/18, C07K 19/00

(54) **TARGETING AGENT**

(30) Priority: 25.04.2022 JP 2022071453
(71) Applicant: Jiksak Bioengineering, Inc., Kawasaki-shi, Kanagawa 210-0821 (JP)
(72) Inventor: YUMOTO, Norihiro, Kawasaki-shi, Kanagawa 210-0821 (JP); INOMATA, Daijiro, Kawasaki-shi, Kanagawa 210-0821 (JP)
(74) Representative: Mewburn Ellis LLP
(86) International application number: PCT/JP2023/016125
(87) International publication number: WO 2023/210585

(57) **Abstract**

A means for targeting a substance to a motor neuron is provided.

A targeting agent to a motor neuron synapse comprising an antibody capable of binding to the intravesicular domain of Synaptotagmin 2 is provided.

## Description

### Technical Field

The present invention relates to a targeting agent to motor neuron, a motor neuron visualizing agent and a composition containing the same, as well as a method for targeting, a method for visualizing motor neuron, a method for preventing or treating, conditions or diseases using the same.

### Background Art

The nerve is composed of central nerves and peripheral nerves, and regulates various emotions, functions of various organs such as muscles and internal organs, and the like. Such neural functions may be impaired due to nerve damage, disease, aging, and the like, and in such cases, mental and physical health may be impaired and may have great effects in the course of social life. Therefore, maintaining and improving neural function is an extremely critical issue because it is directly linked to maintaining and improving the quality of life (QOL).

The central nerves and peripheral nerves are each composed of neurons, which exchange signals with each other via synapse. Synapse is a junction that comprises a cleft formed between the axon terminal of a neuron (the presynapse) and dendrites of another neuron or cells such as skeletal muscles or organs (the postsynapse), and transmits signals through binding of chemicals released from the presynapse to receptors located on the postsynapse. Synaptogenesis (synapse formation) is triggered by the interaction of specific membrane proteins expressed on the presynapse and postsynapse.

In recent years, compounds and peptides capable of maintaining and improving neural function by promoting synaptogenesis have been developed. Patent Literature 1 discloses that certain peptides have a dendritic elongation-promoting function and a synaptogenesis-promoting function in primary cultured cortical neuronal cells (PCN), and that such peptides are used for the treatment of mild cognitive impairment or early dementia. Patent Literature 2 discloses that C-terminal fragment β (CTFβ) generated by cleavage of amyloid precursor protein (APP) by β-secretase promotes synaptogenesis, and that CTFβ is used for treatment of neurodegenerative diseases or the like.

In addition, Patent Literature 3 discloses methods for culturing motor neurons with presynapse using microbeads with an LRRTM molecule or a fusion protein comprising the molecule having immobilized thereon. In this way, it is expected that a number of compounds and drug candidates capable of acting on motor neurons will be provided in the future by developing a technique that can easily screen the influences of drugs on the function of motor neurons in vitro.

### Citation List

### Patent Literature

Patent Literature 1: JP 2012-092048 A
Patent Literature 2: JP 2008-143867 A
Patent Literature 3: WO2021/006075

### Summary of Invention

### Technical Problem

In order for such compounds and drugs to act on a motor neuron, controlled transport of the drugs to the target motor neuron is required. In addition, it may be important to be able to identify the location of the target to which the drugs may act. However, to date, the delivery of substances to neurons such as motor neurons and visualization of the delivery site have not been studied.

Therefore, the object of the present invention is to provide a means for targeting a substance to a motor neuron and a means for visualizing a targeting site.

### Solution to Problem

The present inventors were drawn to the expression of a particular protein on the membrane of synaptic vesicle and intensively studied to solve the above problem and found that a desired substance can be targeted to a motor neuron by using an antibody that binds to the intravesicular domain (N-terminal portion) of Synaptotagmin 2. Further, it has been found that the use of an antibody that binds to other membrane proteins of synaptic vesicle has a similar effect, and thus the present invention has been completed.

Therefore, the present invention encompasses the following:
[1] a targeting agent to a motor neuron comprising an antibody capable of binding to the intravesicular domain of a membrane protein present on the synaptic vesicle of a motor neuron.
[2] the targeting agent according to [1], further comprising a label substance and/or a physiologically active substance.
[3] the targeting agent according to [2], comprising a conjugate of the antibody and the label substance and/or physiologically active substance.
[4] the targeting agent according to any of [1]-[3], wherein the membrane protein is a human-derived protein.
[5] the targeting agent according to any of [1]-[4], wherein the membrane protein comprises any one protein selected from the group consisting of Synaptotagmin 2, Synaptic vesicle glycoprotein 2A, Synaptogyrin 1, Synaptophysin, and Synaptotagmin 1.
[6] the targeting agent according to any of [1] to [5], wherein the intravesicular domain is the domain comprising 4 or more amino acids.
[7] the targeting agent according to [5] or [6], wherein the intravesicular domain consists of the amino acid sequence of any one selected from the group consisting of SEQ ID NOs: 3, 7-12, 15, 16, 19, 20, and 23; the amino acid sequence having addition, deletion, and/or substitution of one or multiple amino acid in amino acid sequence of any one selected from the group consisting of SEQ ID NOs: 3, 7-12, 15, 16, 19, 20, and 23; the amino acid sequence having the sequence identity of 90% or more to the amino acid sequence of any one selected from the group consisting of SEQ ID NOs: 3, 7-12, 15, 16, 19, 20, and 23.
[8] the targeting agent according to any of [2]-[7], wherein the label substance is a fluorescent molecule.
[9] the targeting agent according to any of [2]-[8] comprising the label substance, wherein the targeting agent is a motor neuron visualizing agent.
[10] the targeting agent according to any of [2]-[9], wherein the physiologically active substance is one or more selected from the group consisting of a synaptogenesis-promoting agent, a synapse-maintaining agent, a muscle-enhancing agent, and a neuronal function-modifying agent.
[11] the targeting agent according to any of [1]-[10], wherein the targeting agent is incorporated intracellularly by endocytosis.
[12] a pharmaceutical composition comprising the targeting agent according to any of [2]-[11].
[13] a method for targeting the label substance and/or physiologically active substance to a motor neuron comprising a step of contacting the targeting agent according to any of [2]-[11] and/or the pharmaceutical composition according to [12] with a motor neuron; and a step of delivering the targeting agent and/or pharmaceutical composition to a synapse of the motor neuron.
[14] a method for visualizing a motor neuron comprising a step of contacting the motor neuron visualizing agent according to [9] with a motor neuron; a step of delivering the motor neuron visualizing agent to a synapse of the motor neuron; and a step of detecting a signal of the label substance.
[15] a method for preventing or treating a condition or a disease comprising a step of contacting the targeting agent according to any of [2]-[11] with a motor neuron; and a step of delivering the targeting agent to a synapse of the motor neuron.
[16] the method for preventing or treating according to [15], wherein the condition or disease is a condition or disease exhibiting impaired neural function.
[17] the method for preventing or treating according to [15] or [18], wherein the condition or disease is a neurological disease and a neuromuscular disease.
[18] the method for preventing or treating according to any of [15] to [17], wherein the targeting agent comprises a conjugate of the antibody and physiologically active substance.
[19] use of an antibody capable of binding to the intravesicular domain of a membrane protein present on the synaptic vesicle of a motor neuron in the preparation of a medicament comprising the antibody and physiologically active substance.
[20] a composition for targeting to a motor neuron comprising an antibody capable of binding to the intravesicular domain of a membrane protein present on the synaptic vesicle of a motor neuron.
[21] an antibody capable of binding to the intravesicular domain of a membrane protein present on the synaptic vesicle of a motor neuron for use in a method for preventing or treating a condition or a disease.
[22] an antibody capable of binding to the intravesicular domain of a membrane protein present on the synaptic vesicle of a motor neuron for use in method for targeting a label substance and/or a physiologically active substance to motor neuron.

The present specification encompasses the contents disclosed in Japanese Patent Application No. 2022-071453 on which the priority of the present application is based. Advantageous Effects of Invention

With the targeting agent and method for targeting according to the present invention, the desired substance can be targeted to motor neuron using an antibody. Using an antibody the desired substance can be delivered to a motor neuron (e.g., synaptic vesicle within the cell). Where the substance is a physiologically active substance or a therapeutic agent, conditions and/or diseases due to motor neuron disorders can be treated. Additionally, where the substance is a label substance, a motor neuron can be visualized.

### Brief Description of Drawings

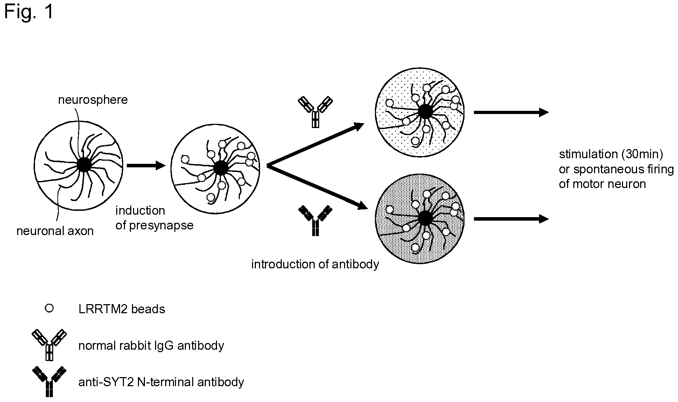

[Figure 1] Figure 1 is a schematic diagram illustrating the experimental procedure for presynapse induction using LRRTM2 beads and antibody delivery. When a neurosphere is seeded on the plate, the neuronal axons are elongated from the neurosphere. By subsequently seeding LRRTM2 beads therein, presynapse is induced from the elongated neuronal axon to the surface of the LRRTM2 bead. On the plate in which presynapse was induced in this way, a normal rabbit IgG antibody (control) or an anti-Synaptotagmin 2 intravesicular domain (N-terminal) antibody (anti-SYT2 N-terminal antibody) is introduced to examine whether the anti-SYT2 N-terminal antibody can be targeted to the presynapse by stimulating neurons with chemicals or spontaneously.

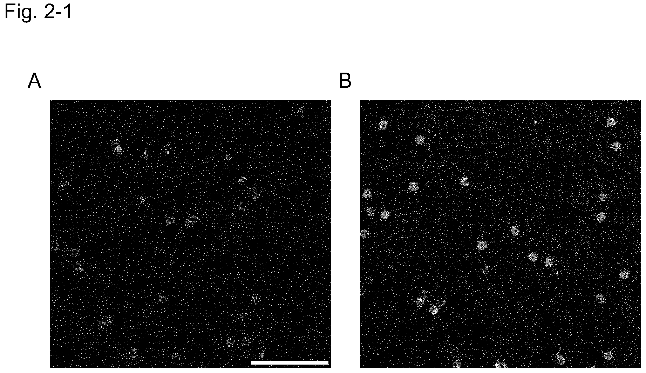

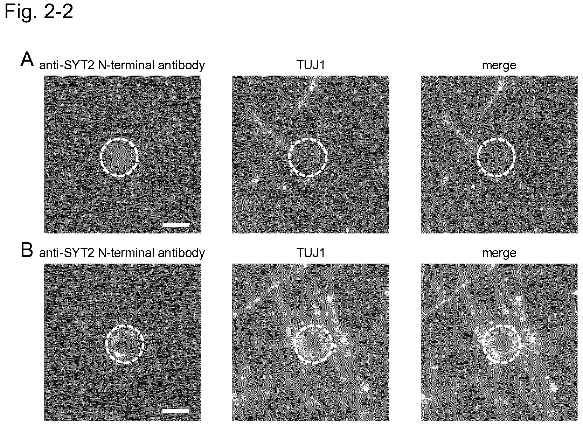

[Figure 2-1] Figure 2-1 shows fluorescent images of the experiment of antibody delivery by spontaneous activity performed at an antibody concentration of 1µg/mL. A and B are fluorescent images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, the scale bar is shared among A and B and represents 100µm.
[Figure 2-2] Figure 2-2 shows fluorescent images of an experimental delivery of an anti-Synaptotagmin 2 intravesicular domain (N-terminal) antibody (anti-SYT2 N-terminal antibody) by spontaneous activity at an antibody concentration of 1µg/mL. A and B are fluorescent images showing the localization of anti-SYT2 N-terminal antibody on an LRRTM2 bead onto which neuronal axons are not densely located (A) and on an LRRTM2 bead onto which neuronal axons are densely located (B), respectively. In the figures, the dashed circles indicate the positions of LRRTM2 beads. TUJ1 refers to stained images showing the signal of βIII-tubulin, and the scale bar is shared among A and B and represents 10µm.

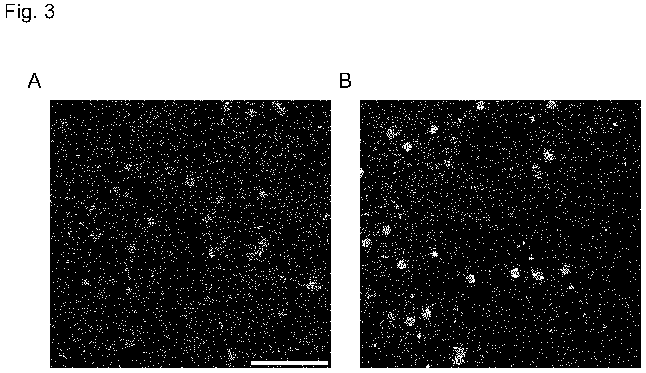

[Figure 3] Figure 3 shows fluorescent images of the experiment of antibody delivery by spontaneous activity performed at an antibody concentration of 10µg/mL. A and B are fluorescent images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, the scale bar is shared among A and B and represents 100µm.

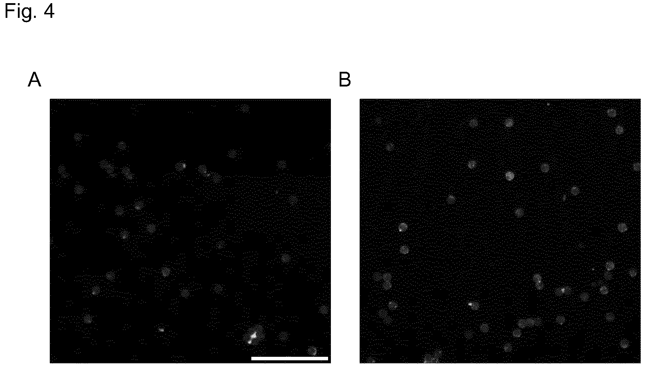

[Figure 4] Figure 4 shows fluorescent images of the experiment of antibody delivery by stimulating neurons with 4-aminopyridine for 10 minutes. A and B are fluorescent images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, the scale bar is shared among A and B and represents 100µm.

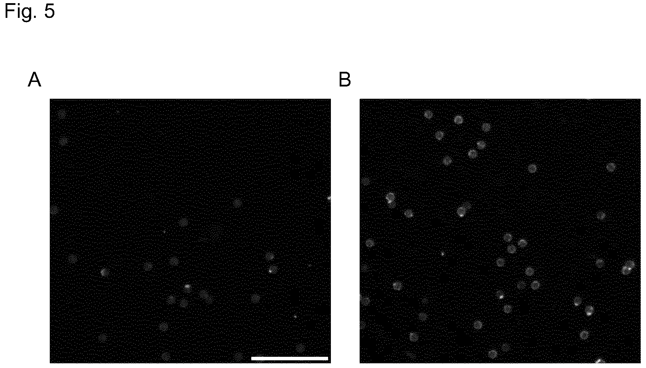

[Figure 5] Figure 5 shows fluorescent images of the experiment of antibody delivery by stimulating neurons with 4-aminopyridine for 30 minutes. A and B are fluorescent images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, the scale bar is shared among A and B and represents 100µm.

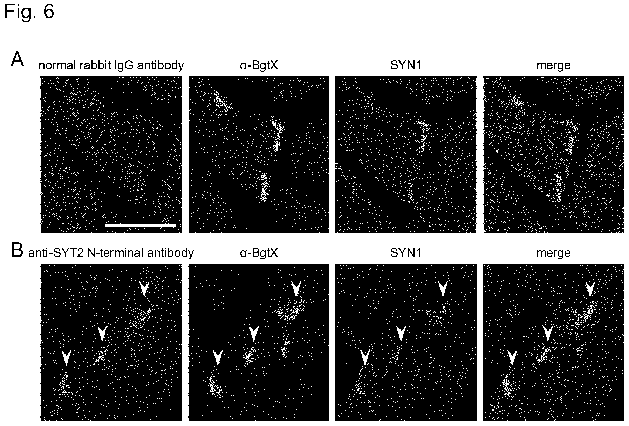

[Figure 6] Figure 6 shows fluorescent images at the neuromuscular junction 12 hours after administration of antibody by tail vein injection. A and B are fluorescent images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, α-BgtX indicates stained images showing a signal of α-bungarotoxin, and SYN1 indicates stained images showing a signal of synapsin 1. The arrowhead indicates the position of the neuromuscular junction to which anti-SYT2 N-terminal antibody was delivered, and the scale bar is shared among each image and represents 50µm.

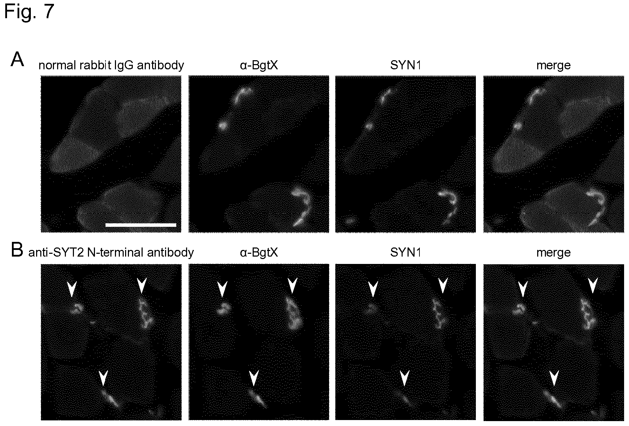

[Figure 7] Figure 7 shows fluorescent images at the neuromuscular junction 72 hours after administration of antibody by tail vein injection. A and B are fluorescent images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, α-BgtX indicates stained images showing a signal of α-bungarotoxin, and SYN1 indicates stained images showing a signal of synapsin 1. The arrowhead indicates the position of the neuromuscular junction to which anti-SYT2 N-terminal antibody was delivered, and the scale bar is shared among each image and represents 50µm.

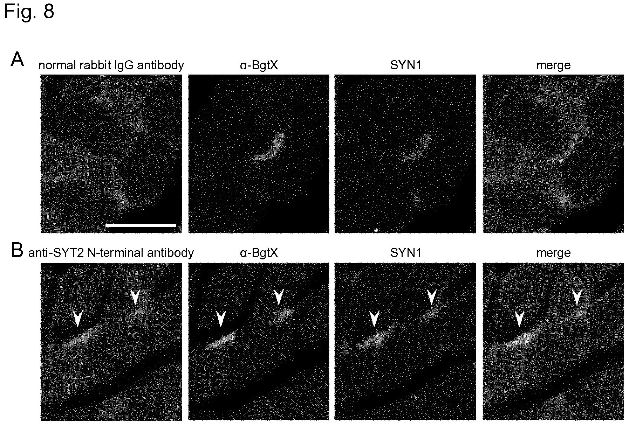

[Figure 8] Figure 8 shows fluorescent images at the neuromuscular junction 12 hours after administration of antibody by intraperitoneal injection. A and B are fluorescent images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, α-BgtX indicates stained images showing a signal of α-bungarotoxin, and SYN1 indicates stained images showing a signal of synapsin 1. The arrowhead indicates the position of the neuromuscular junction to which anti-SYT2 N-terminal antibody was delivered, and the scale bar is shared among each image and represents 50µm.

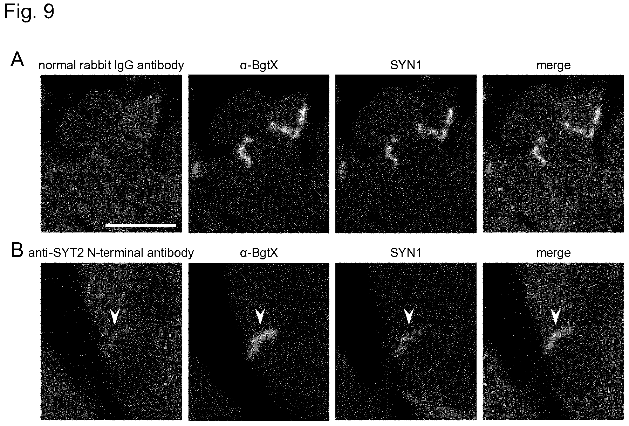

[Figure 9] Figure 9 shows fluorescent images at the neuromuscular junction 72 hours after administration of antibody by intraperitoneal injection. A and B are fluorescent images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, α-BgtX indicates stained images showing a signal of α-bungarotoxin, and SYN1 indicates stained images showing a signal of synapsin 1. The arrowhead indicates the position of the neuromuscular junction to which anti-SYT2 N-terminal antibody was delivered, and the scale bar is shared among each image and represents 50µm.

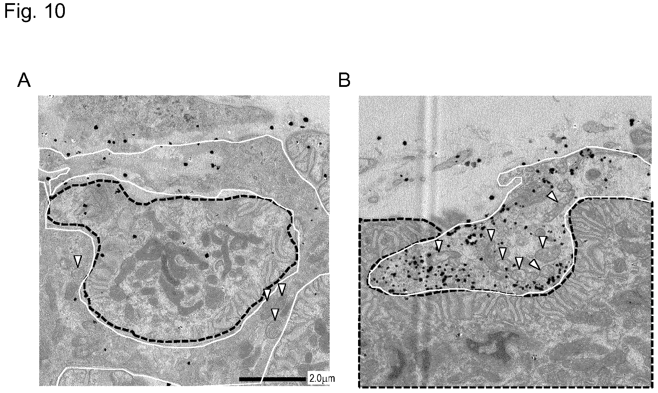

[Figure 10] Figure 10 shows electron-microscopic images at the neuromuscular junction 72 hours after administration of antibody by tail vein injection. A and B are electron-microscopic images showing the localization of applied antibody when a normal rabbit IgG antibody (A) and an anti-Synaptotagmin 2 intravesicular domain (N-terminal) antibody (anti-SYT2 N-terminal antibody) (B) were used as antibody, respectively. In the figure, the area surrounded by the white line indicates the neuronal axon terminal of the motor neuron, and the area surrounded by the black dashed line indicates skeletal muscle cells. Black dots indicate the position of antibody and arrowheads indicate the position of mitochondria. The scale bar is shared among A and B and represents 2µm.

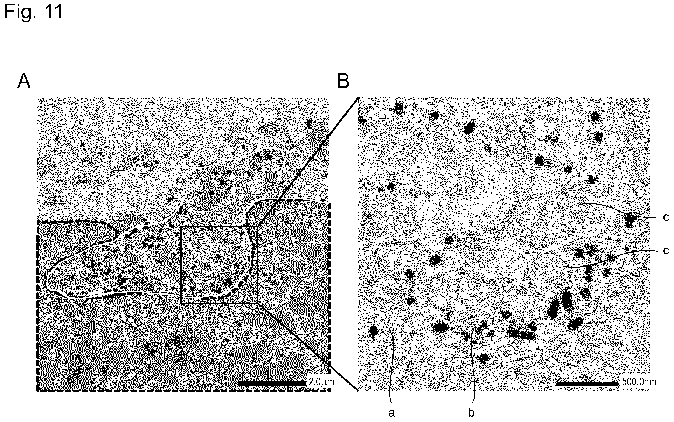

[Figure 11] Figure 11 shows electron-microscopic images at the neuromuscular junction 72 hours after administration of anti-Synaptotagmin 2 intravesicular domain (N-terminal) antibody (anti-SYT2 N-terminal antibody) by tail vein injection. A is an electron microscope image of the same field as Figure 10B, and B is an electron microscope image obtained by enlarging the area surrounded by the black solid line of A. In the figure, the black dots indicate the position of antibody. In Figure A, the area surrounded by the white line indicates the neuronal axon terminal of a motor neuron, the area surrounded by the black dashed line indicates the gastrocnemius muscle cells, which are skeletal muscles, and the scale bar represents 2µm. In Figure B, each of a-c indicates a synaptic vesicle that does not comprise antibody (a), a synaptic vesicle that comprises antibody (b), and mitochondria (c). In Figure B, the scale bar represents 500nm.

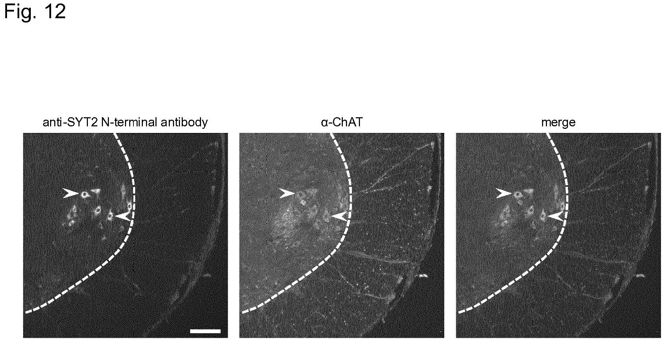

[Figure 12] Figure 12 shows fluorescent images at the spinal cord 72 hours after administration of anti-Synaptotagmin 2 intravesicular domain (N-terminal) antibody (anti-SYT2 N-terminal antibody) by tail vein injection. In the figures, ChAT refers to stained images showing the signal of choline acetyltransferase. The dashed line indicates the border between the anterior horn of the spinal cord and the white matter, and the anterior horn of the spinal cord is located on the left side of the dashed line. The arrowhead illustrates the position of the cell body of motor neuron to which anti-SYT2 N-terminal antibody was delivered, and the scale bar is shared among each image and represents 100µm.

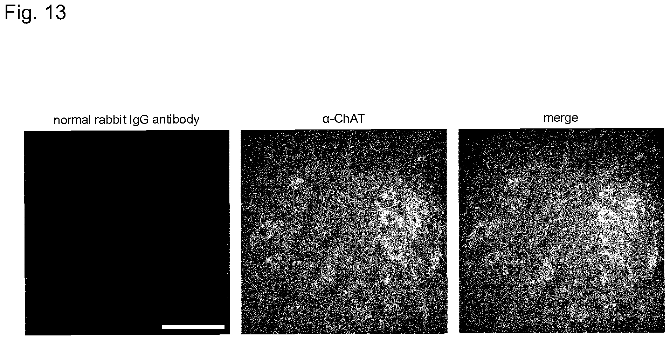

[Figure 13] Figure 13 shows fluorescent images obtained by magnifying the anterior horn of the spinal cord in the spinal cord 72 hours after administration of normal rabbit IgG antibody by tail vein injection. In the figures, ChAT refers to stained images showing the signal of choline acetyltransferase. The scale bar is shared among each image and represents 100µm.

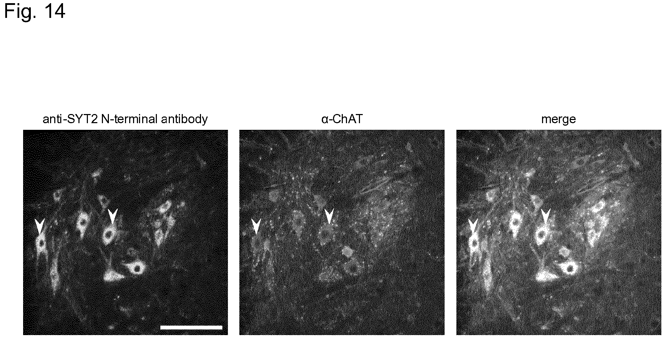

[Figure 14] Figure 14 shows fluorescent images obtained by magnifying the anterior horn of the spinal cord in the spinal cord 72 hours after administration of anti-Synaptotagmin 2 intravesicular domain (N-terminal) antibody (anti-SYT2 N-terminal antibody) by tail vein injection. In the figures, ChAT refers to stained images showing the signal of choline acetyltransferase. The arrowhead illustrates the position of the cell body of motor neuron to which anti-SYT2 N-terminal antibody was delivered, and the scale bar is shared among each image and represents 100µm.

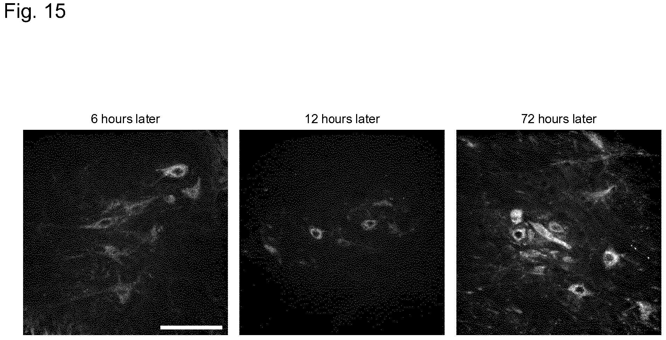

[Figure 15] Figure 15 shows fluorescent images at the anterior horn of the spinal cord from 6 hours to 72 hours after administration of anti-Synaptotagmin 2 intravesicular domain (N-terminal) antibody (anti-SYT2 N-terminal antibody) by tail vein injection. The fluorescent signal shown in the figures is a signal based on anti-SYT2 N-terminal antibody. In the figure, the scale bar is shared among each image and represents 100µm.

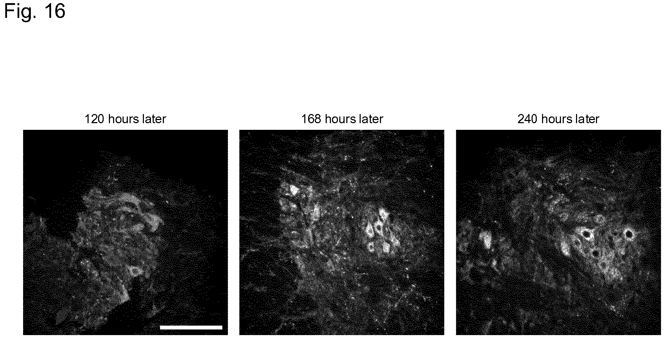

[Figure 16] Figure 16 shows fluorescent images at the anterior horn of the spinal cord from 120 hours to 240 hours after administration of anti-Synaptotagmin 2 intravesicular domain (N-terminal) antibody (anti-SYT2 N-terminal antibody) by tail vein injection. The fluorescent signals shown in the figures are signals based on anti-SYT2 N-terminal antibody. In the figure, the scale bar is shared among each image and represents 100µm.

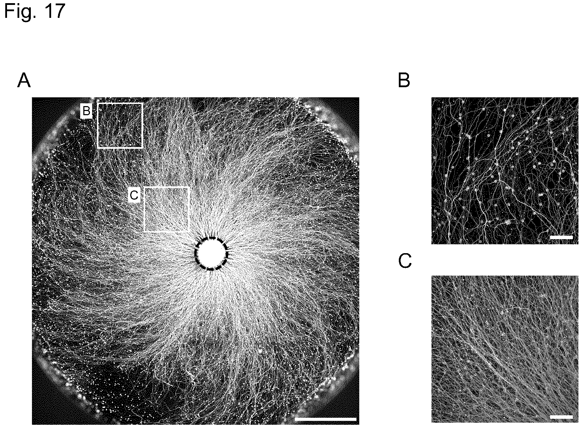

[Figure 17] Figure 17 shows fluorescent images of the control antibody group in which the conjugate of normal rabbit antibody and monomethyl auristatin E (MMAE) was introduced. The fluorescent signal shown in the figure is a signal based on βIII-tubulin (Tuj1). In Figure A, the black dashed circle indicates the position of the neurosphere. Figures B and C show magnified images of the white frame area in Figure A. In Figure A, the scale bar represents 1mm, and in Figures B and C, scale bars represent 100µm.

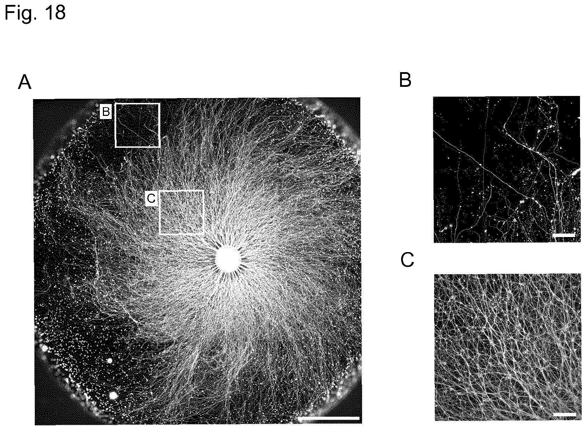

[Figure 18] Figure 18 shows fluorescent images of the SYT2 antibody group in which the conjugate of anti-Synaptotagmin 2 intravesicular domain (N-terminal) antibody (anti-SYT2 N-terminal antibody) and monomethyl auristatin E (MMAE) was introduced. The fluorescent signal shown in the figure is a signal based on βIII-tubulin (Tuj1). Figures B and C show magnified images of the white frame area in Figure A. In the figure, the scale bar represents 1mm, and in Figures B and C, scale bars represent 100µm.

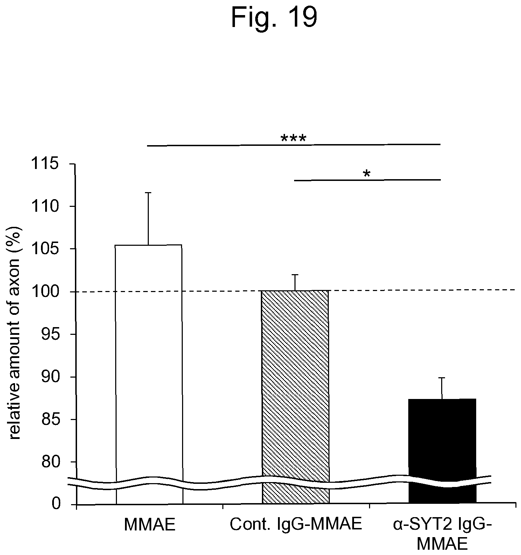

[Figure 19] Figure 19 shows a graph in which the pharmacological effects based on monomethyl auristatin E (MMAE) were quantified. In the figure, the relative amount of axon is the value normalized by the results obtained using the conjugate of the control normal rabbit antibody and MMAE ("Cont. IgG-MMAE" in the figure), set to 100%. In the figure, the dashed line indicates the position where the relative amount of axon is 100%, "MMAE" indicates the result of the simple introduction group in which MMAE was introduced alone, and "α-SYT2 IgG-MMAE" indicates the result of the SYT2 antibody group in which the conjugate of anti-Synaptotagmin 2 intravesicular domain (N-terminal) antibody (anti-SYT2 N-terminal antibody) and monomethyl auristatin E (MMAE) was introduced. In the figure, error bars indicate standard error, "*" indicates p<0.05, and "***" indicates p<0.001.

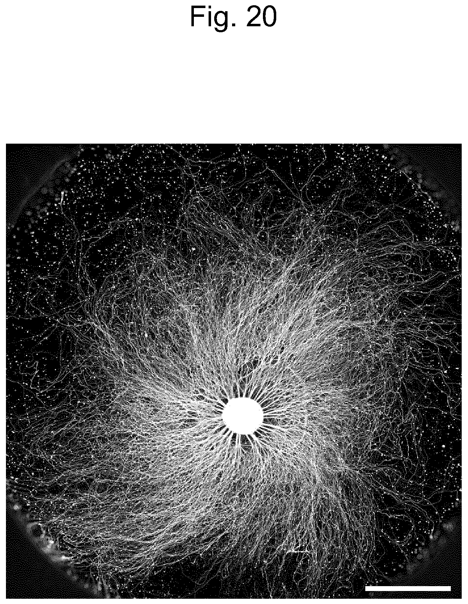

[Figure 20] Figure 20 shows fluorescent images of the normal firing group in which the conjugate of anti-Synaptotagmin 2 intravesicular domain (N-terminal) antibody (anti-SYT2 N-terminal antibody) and monomethyl auristatin E (MMAE) was introduced and neurons were cultured under the same conditions as in Figure 18. The fluorescent signal shown in the figure is a signal based on βIII-tubulin (Tuj1). In the figure, the scale bar represents 1mm.

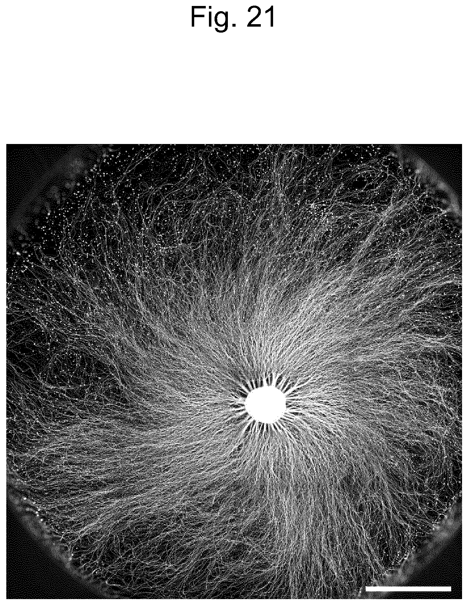

[Figure 21] Figure 21 shows fluorescent images in a synapse non-forming group in which the conjugate of anti-Synaptotagmin 2 intravesicular domain (N-terminal) antibody (anti-SYT2 N-terminal antibody) and monomethyl auristatin E (MMAE) was introduced, and neurons were cultured under the condition not to induce the formation of the presynapse. The fluorescent signal shown in the figure is a signal based on βIII-tubulin (Tuj1). In the figure, the scale bar represents 1mm.

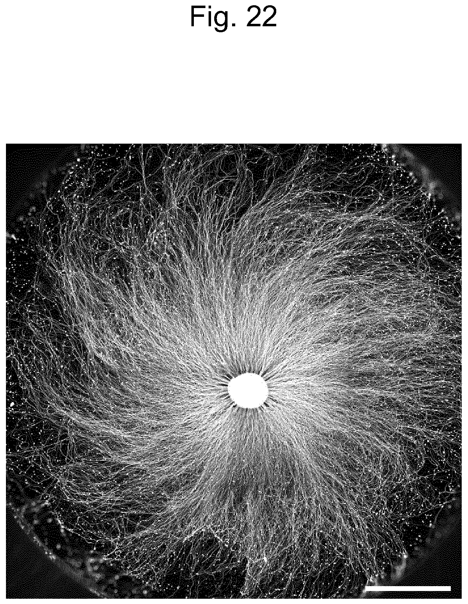

[Figure 22] Figure 22 shows fluorescent images of the endocytosis-inhibiting group in which the conjugate of anti-Synaptotagmin 2 intravesicular domain (N-terminal) antibody (anti-SYT2 N-terminal antibody) and monomethyl auristatin E (MMAE) was introduced, and neurons were cultured under low-temperature conditions in which endocytosis was inhibited. The fluorescent signal shown in the figure is a signal based on βIII-tubulin (Tuj1). In the figure, the scale bar represents 1mm.

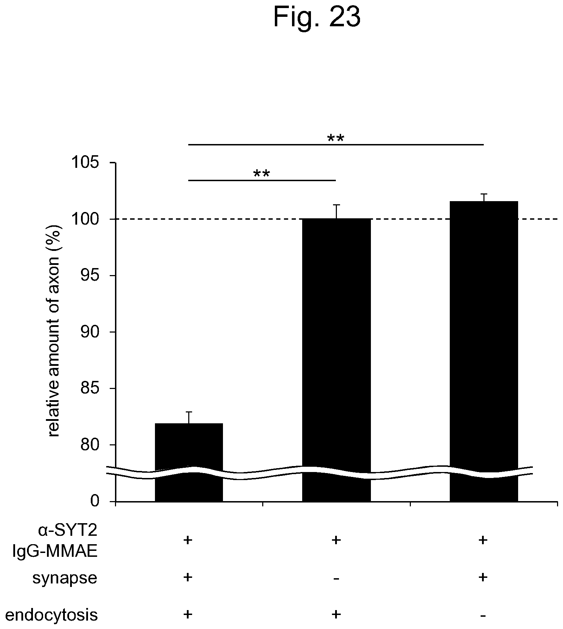

[Figure 23] Figure 23 shows a graph in which the pharmacological effects based on monomethyl auristatin E (MMAE) delivered by anti-Synaptotagmin 2 intravesicular domain (N-terminal) antibody (anti-SYT2 N-terminal antibody) were quantified. In the figure, the relative amount of axon is the value normalized by the synapse non-forming group in which neurons were cultured under the condition not to induce the formation of presynapse, set to 100%. In the figure, the dashed line indicates the position where the relative amount of axon is 100%, "α-SYT2 IgG-MMAE" indicates the presence or absence of introduction of the conjugate of anti-SYT2 N-terminal antibody and MMAE, "synapse" indicates the presence or absence of induction of synaptogenesis, and "endocytosis" indicates the presence or absence of endocytosis. In the figure, error bars indicate standard error, and "**" indicates p<0.01.

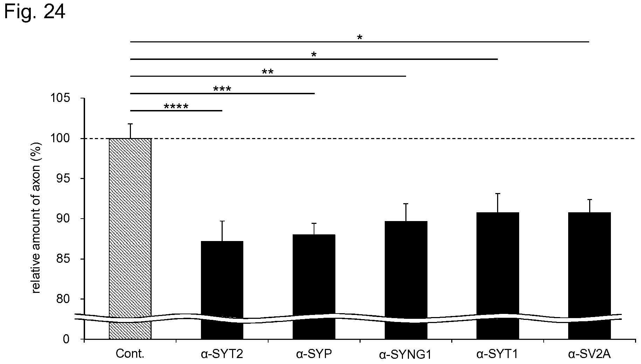

[Figure 24] Figure 24 shows a graph in which the pharmacological effects based on monomethyl auristatin E (MMAE) delivered by each antibody were quantified. The value is being normalized by the results obtained using the conjugate of the control normal rabbit antibody and MMAE ("Cont." in the figure), set to 100%. In the figure, the dashed line indicates the position where the relative amount of axon is 100%, "α-SYT2" indicates the conjugate of anti-Synaptotagmin 2 intravesicular domain (N-terminal) antibody (anti-SYT2 N-terminal antibody) and MMAE, "α-SYP" indicates the conjugate of anti-Synaptophysin intravesicular domain antibody and MMAE, "α-SYNGR1" indicates the conjugate of the anti-Synaptogyrin 1 intravesicular domain antibody and MMAE, "α-SYT1" indicates the conjugate of the anti-Synaptotagmin 1 intravesicular domain antibody and MMAE, and "α-SV2A" indicates the conjugate of the anti-synaptic vesicle protein 2 intravesicular domain antibody and MMAE. In the figure, error bars indicate standard error, "*" indicates p<0.05, "**" indicates p<0.01, "***" indicates p<0.001, and "****" indicates p<0.0001.

### Description of Embodiments

Hereinafter, the present invention is described in detail.

### < Targeting Agent >

The present invention relates to the targeting agent to motor neuron (referred to as the "targeting agent" according to the present invention) comprising an antibody capable of binding to the intravesicular domain of a membrane protein present on the synaptic vesicle of a motor neuron (referred to as "intravesicular domain antibody").

A "membrane protein" refers to a protein present on a biomembrane. "Membrane protein" as used herein refers to a protein present on the membrane of synaptic vesicle, in particular. Membrane protein herein includes any of a transmembrane protein, a membrane extrinsic protein, and a lipid-modified protein. Both membrane extrinsic proteins and lipid-modified proteins are proteins that do not have a transmembrane domain.

The type of membrane protein in the targeting agent according to the present invention is not particularly limited as long as it is a protein comprising an intravesicular domain and is preferably a transmembrane protein.

As used herein, "intravesicular domain" of a protein refers to a protein region in membrane protein that is exposed to the lumen of synaptic vesicle. The length of intravesicular domain of membrane protein in the targeting agent according to the present invention is not particularly limited. For example, a membrane protein having a consecutive intravesicular domain of 1 or more amino acids, 2 or more amino acids, 3 or more amino acids, 4 or more amino acids, 5 or more amino acids, 6 or more amino acids, 7 or more amino acids, 8 or more amino acids, 9 or more amino acids, 10 or more amino acids, or 11 or more amino acids may be used. For example, a transmembrane protein having a consecutive intravesicular domain of 4 amino acids or more can be suitably used.

As used herein, "lumen of synaptic vesicle" refers to the space for the synaptic vesicle that is on the opposite side of the cytoplasm.

The lumen of synaptic vesicle communicates with the extracellular space when synaptic vesicle fuses with the plasma membrane. Therefore, the intravesicular domain in the membrane protein regarding the targeting agent according to the present invention is exposed to the outside of the cell when the synaptic vesicle is fused with the cell membrane.

First, an explanation is provided for the case of using Synaptotagmin 2 as membrane protein as an example.

The present invention relates to the targeting agent to motor neuron comprising an antibody capable of binding to the intravesicular domain (N-terminal portion) of Synaptotagmin 2 (referred to as "anti-SYT2 N-terminal antibody").

As used herein, "Synaptotagmin 2" refers to one of the membrane proteins belonging to the Synaptotagmin family. Synaptotagmin family encompasses 17 proteins in mammals, of which Synaptotagmin 2 is a protein that is expressed primarily on the synaptic vesicle membrane of the presynapse of the neuromuscular junction in peripheral nerves and promotes the fusion of synaptic vesicle with the plasma membrane in a calcium-ion dependent manner (see, for example, Rickman, Colin, et al., Journal of Biological Chemistry 279.13 (2004): 12574-12579., Stephanie Bauche, et al., Neurol Genet. 2020 Dec 3; 6(6): e534. doi: 10.1212). Synaptotagmin 2 is a single-spanning transmembrane protein and comprises, in order from the N-terminus, an intravesicular domain, a transmembrane domain, and a cytoplasmic domain. It is known that the C-terminal cytoplasmic domain has a tandem C2 domain capable of binding calcium ions and that this cytoplasmic domain is mainly responsible for the function related to the fusion of membranes.

Intravesicular domain of Synaptotagmin 2 is exposed to the lumen of synaptic vesicle. When synaptic vesicle and the plasma membrane fuse with each other as the intracellular calcium-ion level increases, the lumen of synaptic vesicle is connected to the extracellular space, and the intravesicular domain of Synaptotagmin 2 is temporarily exposed to the extracellular space. Thereafter, the cellular membrane portion containing Synaptotagmin 2 is recovered as a synaptic vesicle membrane into the cells by endocytosis and reused as a synaptic vesicle. At this time, the intravesicular domain of Synaptotagmin 2 is exposed to the lumen of synaptic vesicle again.

Specifically, an exemplary human Synaptotagmin 2 is a protein consisting of 419 amino acids with an amino acid sequence of SEQ ID NO: 1. For example, in SEQ ID NO: 1, the position of each domain is as follows: the intravesicular domain is a region having an amino acid sequence from position 1 to position 62; the transmembrane domain is a region having an amino acid sequence from position 63 to position 83; and the cytoplasmic domain is a region having an amino acid sequence from position 84 to position 419.

An exemplary mouse Synaptotagmin 2 is a protein consisting of 422 amino acids with an amino acid sequence of SEQ ID NO: 2. In SEQ ID NO: 2, the position of each domain is as follows: intravesicular domain is a region having an amino acid sequence from position 1 to position 60; the transmembrane domain is a region having an amino acid sequence from position 61 to position 87; and the cytoplasmic domain is a region having an amino acid sequence from position 88 to position 422.

The sequence information of Synaptotagmin 2 of other organisms can readily be obtained from known databases such as NCBI databases.

As used herein, "intravesicular domain of Synaptotagmin 2" refers to the entire or a segment of the intravesicular domain present in the N-terminal portion of Synaptotagmin 2. The entire intravesicular domain includes, for example, a region having an amino acid sequence from position 1 to position 62 in SEQ ID NO: 1 (SEQ ID NO: 3), and a region having an amino acid sequence from position 1 to position 60 in SEQ ID NO: 2 (SEQ ID NO: 4). Additionally, a segment of the intravesicular domain includes, for example, a region having a partial sequence with any length from SEQ ID NOs: 3 and 4. More specifically, for example, the regions having an amino acid sequence from position 1 to position 11 in amino acid sequence of SEQ ID NOs: 3 and 4 (both identical to SEQ ID NO: 5), and the N-terminal portion of Synaptotagmin 2 having the mutant amino acid sequence with substitution, insertion, deletion, and/or addition of one or multiple amino acids in an amino acid sequence of SEQ ID NO: 5 are included. "Multiple" herein refers to the number of two or more, for example, 2-6, 2-5, 2-4, or 2-3, and it preferably refers to two.

For the targeting agent according to the present invention, an antibody capable of binding to any membrane protein other than Synaptotagmin 2 exemplified above may be used.

Specific membrane proteins for the targeting agent according to the present invention include, in addition to Synaptotagmin 2, for example, proteins belonging to any one family selected from the group consisting of Synaptotagmin family, Major facilitator superfamily, Synaptic vesicle glycoprotein 2 family, Synaptogyrin family, Synaptophysin/Synaptobrevin family, Synaptobrevin family, vesicular amine transporter family, Solute carrier family 5, Vesicular glutamate transporter family, Amino acid/polyamine transporter family, Secretory carrier membrane protein family, Glutamate transporter subfamily, Autophagy-related protein 9 family, Sugar transporter family, Vacuolar ATPase subunit S1 family.

More specifically, membrane protein for the targeting agent according to the present invention includes, in addition to Synaptotagmin 2, for example, Synaptic vesicle glycoprotein 2A, Synaptogyrin 1, Synaptophysin, Synaptotagmin 1, Synaptogyrin 3, Vesicular acetylcholine transporter, high-affinity choline transporter, Vesicular glutamate transporter 1, Vesicular glutamate transporter 3, Vesicular GABA transporter, Synaptic vesicle glycoprotein 2B, Synaptic vesicle glycoprotein 2C, Vesicle-associated membrane protein 1, Synaptogyrin 4, Synaptotagmin 4, Synaptotagmin 7, Secretory carrier membrane protein 5, Synaptic vesicle 2-related protein (SVOP), Excitatory amino acid transporter 3, Autophagy-related protein 9A, Glucose transporter type 4, and ATPase H+ transporting accessory protein 1. Preferably, membrane protein for the targeting agent according to the present invention comprises any one selected from the group consisting of Synaptotagmin 2, Synaptic vesicle glycoprotein 2A, Synaptogyrin 1, Synaptophysin and Synaptotagmin 1. In this context, the present invention provides the targeting agent comprising an antibody capable of binding to the intravesicular domain of any one of the proteins selected from the group consisting of Synaptotagmin 2, Synaptic vesicle glycoprotein 2A, Synaptogyrin 1, Synaptophysin, and Synaptotagmin 1.

"Synaptic vesicle glycoprotein 2A (SV2A)" refers to one of the 12-spanning transmembrane proteins belonging to Synaptic vesicle glycoprotein 2 family of Major facilitator superfamily. This protein is thought to be involved in the control of regulatory secretion for neurons and endocrine cells as well as to promote infrequent neurotransmission of neurons in the resting phase. Synaptic vesicle glycoprotein 2A is also known as KIAA0736, SV2, SLC22B1, or the like. Exemplary Synaptic vesicle glycoprotein 2A is a human-derived protein consisting of 742 amino acids with an amino acid sequence of SEQ ID NO: 6.

As used herein, " intravesicular domain of Synaptic vesicle glycoprotein 2A" refers to the entire or a segment of the intravesicular domain of Synaptic vesicle glycoprotein 2A. The entire intravesicular domain includes, for example, in SEQ ID NO: 6, a region having an amino acid sequence from position 191 to position 205 (sequence: PSAEKDMCLSDSNKG; SEQ ID NO: 7), a region having an amino acid sequence from position 255 to position 262 (sequence: YGTFLFCR; SEQ ID NO: 8), a region having an amino acid sequence from position 316 to position 334 (sequence: PHYGWSFQMGSAYQFHSWR; SEQ ID NO: 9), a region having an amino acid sequence from position 469 to position 598 (sequence: PDMIRHLQAVDYASRTKVFPGERVEHVTFNFTLENQIHRGGQYFNDKFIGLRLKSVSF EDSLFEECYFEDVTSSNTFFRNCTFINTVFYNTDLFEYKFVNSRLINSTFLHNKEGCPLD VTGTGEGAYMVY; SEQ ID NO: 10), a region having an amino acid sequence from position 648 to position 651 (sequence: ESAM; SEQ ID NO: 11), and a region having an amino acid sequence from position 709 to position 712 (sequence: GITK; SEQ ID NO: 12). Additionally, a segment of the intravesicular domain includes, for example, a region having a partial sequence with any length from SEQ ID NOs: 7-12. The length of the partial sequence is in accordance with the description for the anti-SYT2 N-terminal antibody. More specifically, for example, the regions having an amino acid sequence from position 451 to position 550 in SEQ ID NO: 6 (SEQ ID NO: 13: MGVWFTMSFSYYGLTVWFPDMIRHLQA VDY ASRTKVFPGERVEHVTFNFTLENQIH RGGQYFNDKFIGLRLKSVSFEDSLFEECYFEDVTSSNTFFRNCT), and the protein region of Synaptic vesicle glycoprotein 2A having the mutant amino acid sequence with substitution, insertion, deletion, and/or addition of one or multiple amino acids in an amino acid sequence of SEQ ID NO: 13 are included.

"Synaptogyrin 1 (SYNGR1)" refers to one of the 4-spanning transmembrane proteins belonging to Synaptogyrin family. This protein is present in the presynaptic vesicle of neurons and is thought to be involved in regulatory exocytosis, the formation and maturation of synaptic vesicle, and synapse plasticity. Exemplary Synaptogyrin 1 is a human-derived protein consisting of 233 amino acids with an amino acid sequence of SEQ ID NO: 14.

As used herein, "intravesicular domain of Synaptogyrin 1" refers to the entire or a segment of the intravesicular domain of Synaptogyrin 1. The entire intravesicular domain includes, for example, in SEQ ID NO: 14, a region having an amino acid sequence from position 45 to position 71 (sequence: NEGYLNSASEGEEFCIYNRNPNACSYG; SEQ ID NO: 15) and a region having an amino acid sequence from position 125 to position 148 (sequence: YLANQWQVSKPKDNPLNEGTDAAR; SEQ ID NO: 16). Additionally, a segment of the intravesicular domain includes, for example, a region having a partial sequence with any length from SEQ ID NOs: 15 and 16. The length of the partial sequence is in accordance with the description for the anti-SYT2 N-terminal antibody. More specifically, for example, the regions having an amino acid sequence from position 130 to position 146 in SEQ ID NO: 14 (SEQ ID NO: 17: WQVSKPKDNPLNEGTDA), and the protein region of Synaptogyrin 1 having the mutant amino acid sequence with substitution, insertion, deletion, and/or addition of one or multiple amino acids in an amino acid sequence of SEQ ID NO: 14 are included.

"Synaptophysin (SYP)" refers to one of the 4-spanning transmembrane proteins belonging to Synaptophysin/Synaptobrevin family. This protein is thought to be involved in the organization of vesicle membrane components, targeting of vesicles to the plasma membrane, the control of synapse plasticity, and the like. Synaptophysin is also known as MRX96, tumor synaptic vesicle proteinaceous P38, MRXSYP, XLID96, or the like. An exemplary Synaptophysin is a human-derived protein consisting of 313 amino acids with an amino acid sequence of SEQ ID NO: 18.

As used herein, "intravesicular domain of Synaptophysin" refers to the entire or a segment of the intravesicular domain of Synaptophysin. The entire intravesicular domain includes, for example, in SEQ ID NO: 18, a region having an amino acid sequence from position 50 to position 106 (sequence: ELQLSVDCANKTESDLSIEVEFEYPFRLHQVYFDAPTCRGGTTKVFLVGDYSSSAEF; SEQ ID NO: 19), and a region having an amino acid sequence from position 162 to position 199 (sequence: KGLSDVKMATDPENIIKEMPVCRQTGNTCKELRDPVTS; SEQ ID NO: 20). Additionally, a segment of the intravesicular domain includes, for example, a region having a partial sequence with any length from SEQ ID NOs: 19 and 20. The length of the partial sequence is in accordance with the description for the anti-SYT2 N-terminal antibody. More specifically, for example, the regions having an amino acid sequence from position 178 to position 190 in SEQ ID NO: 18 (SEQ ID NO: 21: CRQTGNTCKELRD), and the protein region of Synaptophysin having the mutant amino acid sequence with substitution, insertion, deletion, and/or addition of one or multiple amino acids in an amino acid sequence of SEQ ID NO: 21 are included.

"Synaptotagmin 1 (SYT1)" refers to one of the single-spanning transmembrane proteins belonging to Synaptotagmin family. This protein is thought to be one of the proteins that promotes the fusion of synaptic vesicle with the plasma membrane in a calcium-ion dependent manner. Synaptotagmin 1 is also known as P65, SVP65, SYT, BAGOS, or the like. Exemplary Synaptotagmin 1 is a human-derived protein consisting of 422 amino acids with an amino acid sequence of SEQ ID NO: 22.

As used herein, "intravesicular domain of Synaptotagmin 1" refers to the entire or a segment of the intravesicular domain of Synaptotagmin 1. The entire intravesicular domain includes, for example, a region having an amino acid sequence from position 1 to position 57 in SEQ ID NO: 22 (sequence: MVSESHHEALAAPPVTTVATVLPSNATEPASPGEGKEDAFSKLKEKFMNELHKIPLP; SEQ ID NO: 23). Additionally, a segment of the intravesicular domain includes, for example, a region having a partial sequence with any length from SEQ ID NO: 23. The length of the partial sequence is in accordance with the description for the anti-SYT2 N-terminal antibody. More specifically, for example, the regions having an amino acid sequence from position 1 to position 8 in SEQ ID NO: 22 (SEQ ID NO: 24: MVSASRPE), and the N-terminal portion of Synaptotagmin 1 having the mutant amino acid sequence with substitution, insertion, deletion, and/or addition of one or multiple amino acids in an amino acid sequence of SEQ ID NO: 24 are included.

"Synaptogyrin 3 (SYNGR3)" refers to one of the 4-spanning transmembrane proteins belonging to Synaptogyrin family. This protein is thought to be involved in regulatory exocytosis, recycling of dopamine, and the like. Exemplary Synaptogyrin 3 is a human-derived protein consisting of 229 amino acids with an amino acid sequence of SEQ ID NO: 25.

As used herein, "intravesicular domain of Synaptogyrin 3" refers to the entire or a segment of the intravesicular domain of Synaptogyrin 3. The entire intravesicular domain includes, for example, in SEQ ID NO: 25, a region having an amino acid sequence from position 51 to position 69 (sequence: TDSGPELRCVFNGNAGACR; SEQ ID NO: 26) and a region having an amino acid sequence from position 126 to position 147 (sequence: LTNQWQRTAPGPATTQAGDAAR; SEQ ID NO: 27). Additionally, a segment of the intravesicular domain includes, for example, a region having a partial sequence with any length from SEQ ID NOs: 26 and 27. The length of the partial sequence is in accordance with the description for the anti-SYT2 N-terminal antibody.

"Vesicular acetylcholine transporter (VAChT)" refers to one of the 12-spanning transmembrane membrane protein belonging to the vesicular amine transporter family. This protein is a transmembrane protein that transports acetylcholine to secretory vesicles and releases it extracellularly. Vesicular acetylcholine transporter is also known as VACHT, SLC18A3, CMS21, or the like. Specifically, an exemplary Vesicular acetylcholine transporter is a human-derived protein consisting of 532 amino acids with an amino acid sequence of SEQ ID NO: 29.

As used herein, "intravesicular domain of Vesicular acetylcholine transporter" refers to the entire or a segment of the intravesicular domain of the Vesicular acetylcholine transporter. The entire intravesicular domain includes, for example, in SEQ ID NO: 29, a region having an amino acid sequence from position 55 to position 125 (sequence: PIVPDYIAHMRGGGEGPTRTPEVWEPTLPLPTPANASAYTANTSASPTAAWPAGSALR PRYPTESEDVKIG; SEQ ID NO: 30), a region having an amino acid sequence from position 174 to position 182 (sequence: DYATLFAAR; SEQ ID NO: 31), a region having an amino acid sequence from position 235 to position 242 (sequence: LYEFAGKR; SEQ ID NO: 32), a region having an amino acid sequence from position 311 to position 325 (sequence: TIATWMKHTMAASEW; SEQ ID NO: 33), a region having an amino acid sequence from position 378 to position 388 (sequence: RSFAPLVVSLC; SEQ ID NO: 34), and a region having an amino acid sequence from position 444 to position 447 (sequence: LGPI; SEQ ID NO: 35). Additionally, a segment of the intravesicular domain includes, for example, a region having a partial sequence with any length from SEQ ID NOs: 30-35. The length of the partial sequence is in accordance with the description for the anti-SYT2 N-terminal antibody.

"High-affinity choline transporter 1 (CHT1)" refers to one of the 13-spanning transmembrane proteins belonging to Solute carrier family 5. This protein is a sodium ion and chloride ion-dependent transmembrane transporter for the high affinity uptake of choline from outside the cell for acetylcholine synthesis. High-affinity choline transporter is also known as SLC5A7, HCHT, CHT, or the like. Exemplary High-affinity choline transporter is a human-derived protein consisting of 580 amino acids with an amino acid sequence of SEQ ID NO: 36.

As used herein, "intravesicular domain of High-affinity choline transporter 1" refers to the entire or a segment of the intravesicular domain of High-affinity choline transporter 1. The entire intravesicular domain includes, for example, in SEQ ID NO: 36, a region having an amino acid sequence from position 1 to position 6 (sequence: MAFHVE; SEQ ID NO: 37), a region having an amino acid sequence from position 70 to position 81 (sequence: GTAEAVYVPGYG; SEQ ID NO: 38), a region having an amino acid sequence from position 147 to position 164 (sequence: GEMFWAAAIFSALGATISVIIDVDMHIS; SEQ ID NO: 39), a region having an amino acid sequence from position 213 to position 237 (sequence: ADIGFTAVHAKYQKPWLGTVDSSEV; SEQ ID NO: 40), a region having an amino acid sequence from position 296 to position 317 (sequence: ASTDWNQTAYGLPDPKTTEEAD; SEQ ID NO: 41), a region having an amino acid sequence from position 398 to position 406 (sequence: LTKTVYGLW; SEQ ID NO: 42), and a region having an amino acid sequence from position 457 to position 481 (sequence: QPLIFYPGYYPDDNGIYNQKFPFKT; SEQ ID NO: 43). Additionally, a segment of the intravesicular domain includes, for example, a region having a partial sequence with any length from SEQ ID NOs: 37-43. The length of the partial sequence is in accordance with the description for the anti-SYT2 N-terminal antibody.

"Vesicular glutamate transporter 1 (VGLUT1)" refers to one of the 12-spanning transmembrane proteins belonging to Vesicular glutamate transporter family. This protein is thought to be a multifunctional co-transport transporter that transports several types of ions, such as sodium and phosphate, as well as L-glutamic acid and chloride ion. Vesicular glutamate transporter 1 is also known as SLC17A7, BNPI, or the like. Exemplary Vesicular glutamate transporter 1 is a human-derived protein consisting of 560 amino acids with an amino acid sequence of SEQ ID NO: 44.

As used herein, "intravesicular domain of vesicle glutamic acid transporter 1" refers to the entire or a segment of the intravesicular domain of Vesicular glutamate transporter 1. The entire intravesicular domain includes, for example, in SEQ ID NO: 44, a region having an amino acid sequence from position 85 to position 116 (sequence: VAIVSMVNNSTTHRGGHVVVQKAQFSWDPETV; SEQ ID NO: 45), a region having an amino acid sequence from position 162 to position 169 (sequence: PSAARVHY; SEQ ID NO: 46), a region having an amino acid sequence from position 230 to position 236 (sequence: QYSGWSS; SEQ ID NO: 47), a region having an amino acid sequence from position 324 to position 341 (sequence: SQPAYFEEVFGFEISKVG; SEQ ID NO: 48), a region having an amino acid sequence from position 400 to position 401 (sequence: SK), and a region having an amino acid sequence from position 457 to position 469 (sequence: GAMTKHKTREEWQ; SEQ ID NO: 49). Additionally, a segment of the intravesicular domain includes, for example, a region having a partial sequence with any length from SEQ ID NOs: 45-49. The length of the partial sequence is in accordance with the description for the anti-SYT2 N-terminal antibody.

"Vesicular glutamate transporter 3 (VGLUT3)" refers to one of the 10-spanning transmembrane proteins belonging to Vesicular glutamate transporter family. This protein is thought to be a multifunctional uniporter that transports several types of ions, such as sodium and phosphate, as well as L-glutamic acid and chloride ion. Vesicular glutamate transporter 3 is also known as SLC17A8, DFNA25, or the like. Exemplary Vesicular glutamate transporter 3 is a human-derived protein consisting of 589 amino acids with an amino acid sequence of SEQ ID NO: 50.

As used herein, "intravesicular domain of Vesicular glutamate transporter 3" refers to the entire or a segment of the intravesicular domain of Vesicular glutamate transporter 3. The entire intravesicular domain includes, for example, in SEQ ID NO: 50, a region having an amino acid sequence from position 98 to position 130 amino acid sequence (sequence: VAIVEMVNNSTVYVDGKPEIQTAQFNWDPETVG; SEQ ID NO: 51), a region having an amino acid sequence from position 175 to position 182 (sequence: PSAARVHY; SEQ ID NO: 52), a region having an amino acid sequence from position 243 to position 249 (sequence: QYIGWSS; SEQ ID NO: 53), a region having an amino acid sequence from position 336 to position 353 (sequence: SQPAYFEEVFGFAISKVG; SEQ ID NO: 54), a region having an amino acid sequence from position 412 to position 413 (sequence: TK), and a region having an amino acid sequence from position 469 to position 481 (sequence: GAMTRHKTREEWQ; SEQ ID NO: 55). Additionally, a segment of the intravesicular domain includes, for example, a region having a partial sequence with any length from SEQ ID NOs: 51-55. The length of the partial sequence is in accordance with the description for the anti-SYT2 N-terminal antibody.

"Vesicular GABA transporter (VGAT)" refers to one of the 10-spanning transmembrane proteins belonging to Amino acid/polyamine transporter family. This protein is an antiporter that transports 4-aminobutanoic acid or glycine in the cytoplasm into vesicles for secretion from the nerve terminal by exchanging them with protons in vesicles. Vesicular GABA transporter is also known as VIAAT, SLC32A1, BA122O1.1, or the like. Exemplary Vesicular GABA transporter is a human-derived protein consisting of 525 amino acids with an amino acid sequence of SEQ ID NO: 56.

As used herein, "intravesicular domain of Vesicular GABA transporter" refers to the entire or a segment of the intravesicular domain of Vesicular GABA transporter. The entire intravesicular domain includes, for example, in SEQ ID NO: 56, a region having an amino acid sequence from position 154 to position 204 (sequence: FAAVVCCYTGKILIACLYEENEDGEVVRVRDSYVAIANACCAPRFPTLGGR; SEQ ID NO: 57), a region having an amino acid sequence from position 287 to position 305 (sequence: SRARDWAWEKVKFYIDVKK; SEQ ID NO: 58), a region having an amino acid sequence from position 363 to position 383 (sequence: ADETKEVITDNLPGSIRAVVN; SEQ ID NO: 59), a region having an amino acid sequence from position 460 to position 461 (sequence: TG), and a region having an amino acid sequence from position 511 to position 525 (sequence: SLEGLIEAYRTNAED; SEQ ID NO: 60). Additionally, a segment of the intravesicular domain includes, for example, a region having a partial sequence with any length from SEQ ID NOs: 57-60. The length of the partial sequence is in accordance with the description for the anti-SYT2 N-terminal antibody.

"Synaptic vesicle glycoprotein 2B (SV2B)" refers to one of the 12-spanning transmembrane proteins belonging to Synaptic vesicle glycoprotein 2 family of Major facilitator superfamily. This protein has been suggested to be involved in the control of regulatory secretion for neurons and endocrine cells and function as a protein receptor for botulinum neurotoxin E in neurons. Synaptic vesicle glycoprotein 2B is also known as KIAA0735, HsT19680, SLC22B2, or the like. Exemplary Synaptic vesicle glycoprotein 2B is a human-derived protein consisting of 683 amino acids with an amino acid sequence of SEQ ID NO: 61.

As used herein, "intravesicular domain of Synaptic vesicle glycoprotein 2B" refers to the entire or a segment of the intravesicular domain of Synaptic vesicle glycoprotein 2B. The entire intravesicular domain includes, for example, in SEQ ID NO: 61, a region having an amino acid sequence from position 130 to position 148 (sequence: SFALPSAEKDMCLSSSKKG; SEQ ID NO: 62), a region having an amino acid sequence from position 204 to position 205 (sequence: CR), a region having an amino acid sequence from position 259 to position 277 (sequence: PHYGWGFSMGTNYHFHSWR; SEQ ID NO: 63), a region having an amino acid sequence from position 412 to position 535 (sequence: PDMIRYFQDEEYKSKMKVFFGEHVYGATINFTMENQIHQHGKLVNDKFTRMYFKHV LFEDTFFDECYFEDVTS TDTYFKNCTIESTIFYNTDL YEHKFINCRFINSTFLEQKEGCH MDLEQDND; SEQ ID NO: 64), a region having an amino acid sequence from position 587 to position 592 (sequence: NSESAM; SEQ ID NO: 65), and a region having an amino acid sequence from position 650 to position 653 (sequence: GITK; SEQ ID NO: 66). Additionally, a segment of the intravesicular domain includes, for example, a region having a partial sequence with any length from SEQ ID NOs: 62-66. The length of the partial sequence is in accordance with the description for the anti-SYT2 N-terminal antibody.

"Synaptic vesicle glycoprotein 2C (SV2C)" refers to one of the 12-spanning transmembrane proteins belonging to Synaptic vesicle glycoprotein 2 family of Major facilitator superfamily. This protein is thought to be involved in the control of regulatory secretion for neurons and endocrine cells as well as in neurotransmitter transport and transmembrane transport. Synaptic vesicle glycoprotein 2C is also known as SLC22B3, KIAA1054, or the like. Exemplary Synaptic vesicle glycoprotein 2C is a human-derived protein consisting of 727 amino acids with an amino acid sequence of SEQ ID NO: 67.

As used herein, "intravesicular domain of Synaptic vesicle glycoprotein 2C" refers to the entire or a segment of the intravesicular domain of Synaptic vesicle glycoprotein 2C. The entire intravesicular domain includes, for example, in SEQ ID NO: 67, a region having an amino acid sequence from position 176 to position 191 (sequence: LPSAETDLCIPNSGSG; SEQ ID NO: 68), a region having an amino acid sequence at position 248 (sequence: R), a region having an amino acid sequence from position 302 to position 320 (sequence: PHYGWSFSMGSAYQFHSWR; SEQ ID NO: 69), a region having an amino acid sequence from position 459 to position 578 (sequence: KPLQSDEYALLTRNVERDKYANFTINFTMENQIHTGMEYDNGRFIGVKFKSVTFKDS VFKSCTFEDVTSVNTYFKNCTFIDTVFDNTDFEPYKFIDSEFKNCSFFHNKTGCQITFD DDYS; SEQ ID NO: 70), a region having an amino acid sequence from position 631 to position 636 (sequence: TSESMM; SEQ ID NO: 71), and a region having an amino acid sequence from position 691 to position 698 (sequence: SLVSITKS; SEQ ID NO: 72). Additionally, a segment of the intravesicular domain includes, for example, a region having a partial sequence with any length from SEQ ID NOs: 68-72. The length of the partial sequence is in accordance with the description for the anti-SYT2 N-terminal antibody.

"Vesicle-associated membrane protein 1(VAMP1)" refers to one of the single-spanning transmembrane proteins belonging to Synaptobrevin family. This protein is thought to be involved in targeting and/or membrane fusion of transport vesicles to cell membranes and the like. Vesicle-associated membrane protein 1 is also known as SYB1, CMS25, SPAX1, Synaptobrevin 1, or the like. Exemplary Vesicle-associated membrane protein 1 is a human-derived protein consisting of 118 amino acids with an amino acid sequence of SEQ ID NO: 73.

As used herein, "intravesicular domain of Vesicle-associated membrane protein 1" refers to the entire or a segment of the intravesicular domain of Vesicle-associated membrane protein 1. The entire intravesicular domain includes, for example, a region having an amino acid sequence from position 117 to position 118 in SEQ ID NO: 73 (sequence: FT).

"Synaptogyrin 4 (SYNGR4)" refers to one of the 4-spanning transmembrane proteins belonging to Synaptogyrin family. This protein has 4 transmembrane regions similar to other proteins of the same family. Exemplary Synaptogyrin 4 is a human-derived protein consisting of 234 amino acids with an amino acid sequence of SEQ ID NO: 74.

As used herein, "intravesicular domain of Synaptogyrin 4" refers to the entire or a segment of the intravesicular domain of Synaptogyrin 4. The entire intravesicular domain includes, for example, in SEQ ID NO: 74, a region having an amino acid sequence from position 46 to position 65 (sequence: YQNKMESPQLHCILNSNSVA; SEQ ID NO: 75) and a region having an amino acid sequence from position 125 to position 144 (sequence: ANQWQHSPPKEFLLGSSSAQ; SEQ ID NO: 76). Additionally, a segment of the intravesicular domain includes, for example, a region having a partial sequence with any length from SEQ ID NOs: 75 and 76. The length of the partial sequence is in accordance with the description for the anti-SYT2 N-terminal antibody.

"Synaptotagmin 4 (SYT4)" refers to one of the single-spanning transmembrane proteins belonging to Synaptotagmin family. This protein is thought to be involved in the trafficking of dense-core vesicle of neurons by interacting with KIF1A. Synaptotagmin 4 is also known as KIAA1342, HsT1192, or the like. Exemplary Synaptotagmin 4 is a human-derived protein consisting of 425 amino acids with an amino acid sequence of SEQ ID NO: 77.

As used herein, "intravesicular domain of Synaptotagmin 4" refers to the entire or a segment of the intravesicular domain of Synaptotagmin 4. The entire intravesicular domain includes, for example, a region having an amino acid sequence from position 1 to position 16 in SEQ ID NO: 77 (sequence: MAPITTSREEFDEIPT; SEQ ID NO: 78). Additionally, a segment of the intravesicular domain includes, for example, a region having a partial sequence with any length from SEQ ID NO: 78. The length of the partial sequence is in accordance with the description for the anti-SYT2 N-terminal antibody.

"Synaptotagmin 7 (SYT7)" refers to one of the single-spanning transmembrane proteins belonging to Synaptotagmin family. This protein is thought to be one of the proteins that promote the fusion of secretory vesicle and synaptic vesicle with the plasma membrane in a calcium-ion-dependent manner. Synaptotagmin 7 is also known as IPCA-7, PCANAP7, MGC150517, or the like. Exemplary Synaptotagmin 7 is a human-derived protein consisting of 403 amino acids with an amino acid sequence of SEQ ID NO: 79.

As used herein, "intravesicular domain of Synaptotagmin 7" refers to the entire or a segment of the intravesicular domain of Synaptotagmin 7. The entire intravesicular domain includes, for example, a region having an amino acid sequence from position 1 to position 16 in SEQ ID NO: 79 (sequence: MYRDPEAASPGAPSRD; SEQ ID NO: 80). Additionally, a segment of the intravesicular domain includes, for example, a region having a partial sequence with any length from SEQ ID NO: 80. The length of the partial sequence is in accordance with the description for the anti-SYT2 N-terminal antibody.

"Secretory carrier membrane protein 5 (SCAMP5)" refers to one of the 4-spanning transmembrane proteins belonging to Secretory carrier membrane protein family. This protein is thought to be involved in calcium-dependent exocytosis of cytokines and the like. Secretory carrier membrane protein 5 is also known as MGC24969, HSCAMP5 and the like. Exemplary Secretory carrier membrane protein 5 is a human-derived protein consisting of 235 amino acids with an amino acid sequence of SEQ ID NO: 81.

As used herein, "intravesicular domain of Secretory carrier membrane protein 5" refers to the entire or a segment of the intravesicular domain of Secretory carrier membrane protein 5. The entire intravesicular domain includes, for example, in SEQ ID NO: 81, a region having an amino acid sequence from position 61 to position 67 (sequence: WLIGGGG; SEQ ID NO: 82) and a region having an amino acid sequence from position 126 to position 148 (sequence: IPGWGVCGWIATISFFGTNIGSA; SEQ ID NO: 83). Additionally, a segment of the intravesicular domain includes, for example, a region having a partial sequence with any length from SEQ ID NOs: 82 and 83. The length of the partial sequence is in accordance with the description for the anti-SYT2 N-terminal antibody.

"Synaptic vesicle 2-related protein (SVOP)" refers to one of the 12-spanning transmembrane proteins belonging to Synaptic vesicle glycoprotein 2 family of Major facilitator superfamilies. This protein is thought to have transmembrane transporter activity. Synaptic vesicle 2-related protein is also known as SLC22B4, DKFZp761H039, SCF22B4, or the like. Exemplary Synaptic vesicle 2-related protein is a human-derived protein consisting of 548 amino acids with an amino acid sequence of SEQ ID NO: 84.

As used herein, "intravesicular domain of Synaptic vesicle 2-related protein" refers to the entire or a segment of the intravesicular domain of Synaptic vesicle 2-related protein. The entire intravesicular domain includes, for example, in SEQ ID NO: 84, a region having an amino acid sequence from position 109 to position 122 (sequence: PQLHCEWRLPSWQV; SEQ ID NO: 85), a region having an amino acid sequence from position 178 to position 180 (sequence: VLR), a region having an amino acid sequence from position 231 to position 238 (sequence: VMPSLGWR; SEQ ID NO: 86), a region having an amino acid sequence from position 338 to position 373 (sequence: TTELFQAGDVCGISSRKKAVEAKCSLACEYLSEEDY; SEQ ID NO: 87), a region having an amino acid sequence from position 423 to position 424 (sequence: RN), and a region having an amino acid sequence from position 479 to position 489 (sequence: AQVMLESSVYL; SEQ ID NO: 88). Additionally, a segment of the intravesicular domain includes, for example, a region having a partial sequence with any length from SEQ ID NOs: 85-88. The length of the partial sequence is in accordance with the description for the anti-SYT2 N-terminal antibody.

"Excitatory amino acid transporter 3 (EAAT3)" refers to one of the 8-spanning transmembrane proteins belonging to Glutamate transporter subfamily. This protein is thought to be a sodium-dependent, high affinity amino acid transporter that mediates the uptake of glutamate, aspartic acid, and cysteine. Excitatory amino acid transporter 3 is also known as SLC1A1, HEAAC1, EAAC1, or the like. Exemplary Excitatory amino acid transporter 3 is a human-derived protein consisting of 524 amino acids with an amino acid sequence of SEQ ID NO: 89.

As used herein, "intravesicular domain of Excitatory amino acid transporter 3" refers to the entire or a segment of the intravesicular domain of Excitatory amino acid transporter 3. The entire intravesicular domain includes, for example, in SEQ ID NO: 89, a region having an amino acid sequence from position 39 to position 61 (sequence: REHSNLSTLEKFYFAFPGEILMR; SEQ ID NO: 90), a region having an amino acid sequence from position 115 to position 205 (sequence: SIKPGVTQKVGEIARTGSTPEVSTVDAMLDLIRNMFPENLVQACFQQYKTKREEVKPP SDPEMNMTEESFTAVMTTAISKNKTKEYKIVGM; SEQ ID NO: 91), a region having an amino acid sequence from position 267 to position 286 (sequence: AGKIIEVEDWEIFRKLGLYM; SEQ ID NO: 92), a region having an amino acid sequence from position 381 to position 393 (sequence: IAQLNDLDLGIGQ; SEQ ID NO: 93), and a region having an amino acid sequence from position 428 to position 440 (sequence: LPAEDVTLIIAVD; SEQ ID NO: 94). Additionally, a segment of the intravesicular domain includes, for example, a region having a partial sequence with any length from SEQ ID NOs: 90-94. The length of the partial sequence is in accordance with the description for the anti-SYT2 N-terminal antibody.

"Autophagy-related protein 9A (ATG9A)" refers to one of the 5-spanning transmembrane proteins belonging to Autophagy-related protein 9 family. This protein is a phospholipid scramblase involved in autophagy by modifying the phospholipid composition on autophagosome membranes and mediating its expansion. Autophagy-related protein 9A is also known as APG9L1, FLJ22169, MATG9, or the like. Exemplary Autophagy-related protein 9A is a human-derived protein consisting of 839 amino acids with an amino acid sequence of SEQ ID NO: 95.

As used herein, "intravesicular domain of Autophagy-related protein 9A" refers to the entire or a segment of the intravesicular domain of Autophagy-related protein 9A. The entire intravesicular domain includes, for example, in SEQ ID NO: 95, a region having an amino acid sequence from position 85 to position 128 (sequence: SCVDYDILFANKMVNHSLHPTEPVKVTLPDAFLPAQVCSARIQE; SEQ ID NO: 96) and a region having an amino acid sequence from position 398 to position 406 (sequence: DEDVLAVEH; SEQ ID NO: 97). Additionally, a segment of the intravesicular domain includes, for example, a region having a partial sequence with any length from SEQ ID NOs: 96 and 97. The length of the partial sequence is in accordance with the description for the anti-SYT2 N-terminal antibody.

"Glucose Transporter type 4 (GLUT4)" refers to one of the 12-spanning transmembrane proteins belonging to Sugar transporter family. This protein is a glucose transporter controlled by insulin and plays an important role in the removal of glucose from the systemic circulation. Glucose transporter 4 is also known as SLC2A4, or the like. Exemplary Glucose transporter type 4 is a human-derived protein consisting of 509 amino acids with an amino acid sequence of SEQ ID NO: 98.

As used herein, "intravesicular domain of Glucose transporter type 4" refers to the entire or a segment of the intravesicular domain of Glucose transporter type 4. The entire intravesicular domain includes, for example, in SEQ ID NO: 98, a region having an amino acid sequence from position 46 to position 81 (sequence: NAPQKVIEQSYNETWLGRQGPEGPSSIPPGTLTTLW; SEQ ID NO: 99), a region having an amino acid sequence from position 133 to position 142 (sequence: ASYEMLILGR; SEQ ID NO: 100), a region having an amino acid sequence from position 193 to position 201 (sequence: ESLLGTASL; SEQ ID NO: 101), a region having an amino acid sequence from position 309 to position 323 (sequence: YSTSIFETAGVGQPA; SEQ ID NO: 102), a region having an amino acid sequence from position 375 to position 384 (sequence: ERVPAMSYVS; SEQ ID NO: 103), and a region having an amino acid sequence from position 439 to position 445 (sequence: QYVAEAM; SEQ ID NO: 104). Additionally, a segment of the intravesicular domain includes, for example, a region having a partial sequence with any length from SEQ ID NOs: 99-104. The length of the partial sequence is in accordance with the description for the anti-SYT2 N-terminal antibody.

"ATPase H+ transporting accessory protein 1 (ATP6AP1)" refers to one of the single transmembrane proteins belonging to Vacuolar ATPase subunit S1 family. This protein is thought to be a subunit of the proton-transporting vacuole (V-type) ATPase protein complex required for acidification of the lumen of secretory vesicles. ATPase H+ transporting accessory protein 1 is also known as VATPS1, XAP3, ATP6IP1, ATP6S1, or the like. Exemplary ATPase H+ transporting accessory protein 1 is a human-derived protein consisting of 470 amino acids with an amino acid sequence of SEQ ID NO: 105.

As used herein, "intravesicular domain of ATPase H+ Transport Accessory Protein 1" refers to the entire or a segment of the intravesicular domain of ATPase H+ Transport Accessory Protein 1. The entire intravesicular domain includes, for example, a region having an amino acid sequence from position 42 to position 419 in SEQ ID NO: 105 (sequence: EQQVPLVLWSSDRDLWAPAADTHEGHITSDLQLSTYLDPALELGPRNVLLFLQDKLSI EDFTAYGGVFGNKQDSAFSNLENALDLAPSSLVLPAVDWYAVSTLTTYLQEKLGASP LHVDLATLRELKLNASLPALLLIRLPYTASSGLMAPREVLTGNDEVIGQVLSTLKSED VPYTAALTAVRPSRVARDVAVVAGGLGRQLLQKQPVSPVIHPPVSYNDTAPRILFWA QNFSVAYKDQWEDLTPLTFGVQELNLTGSFWNDSFARLSLTYERLFGTTVTFKFILAN RLYPVSARHWFTMERLEVHSNGSVAYFNASQVTGPSIYSFHCEYVSSLSKKGSLLVA RTQPSPWQMMLQDFQIQAFNVMGEQFSYASDCA; SEQ ID NO: 106). Additionally, a segment of the intravesicular domain includes, for example, a region having a partial sequence with any length from SEQ ID NO: 106. The length of the partial sequence is in accordance with the description for the anti-SYT2 N-terminal antibody.

Furthermore, an antibody capable of binding to non-protein regions of membrane protein present in the vesicle that are exposed to the lumen of the vesicle may be used for the targeting agent according to the present invention. Such a non-protein region includes, for example, sugar chain, lipid, and the like. In addition to the antibody capable of binding to a sugar chain or the like on an intravesicular domain as described above, for example, if a part of the lipid anchors of a lipid-modified protein without a transmembrane domain is exposed to the vesicle lumen, an antibody capable of binding to the part may also be used. Specifically, for example, an antibody capable of binding to a lipid-anchoring moiety of a Ras-associate protein such as Rab3a (SEQ ID NO: 110) or the like may be used. Additionally, for example, an antibody capable of binding to hydrophobic domains present in the vesicle membrane of a membrane extrinsic protein may be used. For example, an antibody capable of binding to the hydrophobic domain present in the vesicle membrane of Synapsin family proteins such as Synapsin 1 (SYN1: SEQ ID NO: 107), Synapsin 2 (SYN2: SEQ ID NO: 108), Synapsin 3 (SYN3: SEQ ID NO: 109) and the like may be used.

The animal from which the membrane protein in the present specification is derived is not particularly limited, and the protein may be derived from various vertebrates and mammals described below with respect to cells and is preferably a protein derived from a human.

As used herein, "targeting agent" refers to a drug for delivering a particular substance to a target. In the present specification, by using the targeting agent, a desired substance (label substance and/or physiologically active substance) is transported to a motor neuron, in particular a motor neuron synapse. In addition, in targeting to a synapse and synaptic vesicle, delivery to the cell body is achieved through incorporation into a cell, particularly into a synaptic vesicle, as the synaptic vesicle is recovered via endocytosis. Furthermore, the targeting agent according to the present invention allows the transported substance to exert physiological activity in the cytoplasm.

For example, the transported desired substance (label substance and/or physiologically active substance) may penetrate the synaptic vesicle membrane and migrate into the cytoplasm upon the targeting agent according to the present invention is incorporated into a synaptic vesicle. Specifically, for example, where a physiologically active substance is used, at least one physiologically active substance in the targeting agent may migrate to the cytoplasm and act on a biological substance in the nucleus, on a desired biological substance in the cytoplasm, or on a biological substance on the cytoplasmic membrane. The biological substance to which a physiologically active substance acts is not particularly limited as long as it can be present on a cell membrane or in a cell, and may be, for example, any of a polymeric compound such as a protein or a nucleic acid, a low molecular weight compound such as a lipid, a sugar, an amino acid, a nucleotide, and the like, and an ion such as a metal ion, an atom, or the like.

Specifically, for example, a biological substance in the nucleus includes DNA, RNA (such as mRNA, siRNA, miRNA), transcription factor, nuclear receptor, and the like, and a biological substance in the cytoplasm includes, in addition to said nucleic acids, cytoskeleton, enzyme, metal ion, and the like. For example, a biological substance on the cell membrane includes lipid of the cell membrane, receptor on the cell membrane, enzyme coupled with the receptor, cell adhesion factor, and the like.

As used herein, "motor neuron" refers to a group of neurons that transmit central stimuli to the effector, a skeletal muscle. Although a motor neuron generally includes a primary motor neuron that is a central neuron and a secondary motor neuron that is a peripheral neuron, the motor neuron of the present specification is a secondary motor neuron.

As used herein, "secondary motor neuron" refers to a motor neuron that has a cell body in the anterior horn of the spinal cord or brain stem and projects the axon to the junction with skeletal muscles. For example, a motor neuron in the present specification includes α motor neuron, β motor neuron, γ motor neuron, and the like. In addition to spinal neurons having a cell body in the anterior nucleus of the spinal cord, some cranial nerves, such as the oculomotor nerve, trochlear nerve, abducens nerve, facial nerve, and sublingual nerve, are also included. As used herein, motor neuron normally secretes acetylcholine as a neurotransmitter and is classified as a cholinergic neuron. However, a motor neuron that secretes a neurotransmitter other than acetylcholine may also be used. The muscle cell projected by the motor neuron herein is a skeletal muscle cell.

As used herein, "skeletal muscle cell" refers to a cell constituting a striated muscle that moves the skeleton or a cell having this phenotype. Skeletal muscle cell herein broadly includes a muscle cell attached to bone and other muscle cells comprised in skeletal muscle, such as a muscle spindle. The type of skeletal muscle is not particularly limited, and examples thereof include the diaphragm muscle, vastus lateralis muscle, vastus medialis muscle, rectus femoris muscle, vastus intermedius muscle, biceps brachii muscle, tibialis anterior muscle, tibialis posterior muscle, gastrocnemius muscle, soleus muscle, deltoid muscle, latissimus dorsi muscle, sternocleidomastoid muscle, intercostal muscle, ocular muscle, facial muscle, tongue muscle, stapedial muscle, and the like. The skeletal muscle cell in the present specification also includes a cultured skeletal muscle cell such as a cell differentiated in vitro from an artificial stem cell (such as an iPS cell and an ES cell) and/or a natural stem cell (such as a mesenchymal stem cell and a skeletal muscle stem cell).

As used herein, a cell may be a cell derived from a vertebrate. Vertebrates include fish, reptiles, amphibians, birds and mammals. Specific mammals include, for example, primates (e.g., humans). The cell may also be a cell derived from domestic animals (such as chickens, horses, cows, sheep, goats, pigs), pet animals (such as tropical fish, lizards, dogs, cats, rabbits), laboratory animals (such as frogs, mice, rats, monkeys). The cell may not be derived from a single tissue, individual, or animal species, and may be a mixture of multiple types of cells. In addition, the health condition of the tissue and the individual from which the cell is derived is not particularly limited.

According to the targeting agent according to the present invention, the desired substance (label substance and/or physiologically active substance) can be targeted to motor neuron (e.g., motor neuron axon terminal, axon, axon hillock, cell body, dendrite, and the like) via motor neuron synapse.

As used herein, "synapse" refers to a junction comprising a cleft formed between an axon terminal of a neuron and a dendrite of another neuron (in the case of the central nervous system) or a cell of skeletal muscle, organ, or the like (in the case of the peripheral nervous system).

As used herein, synapse may be a chemical synapse, such as an excitatory synapse, an inhibitory synapse, or the like. As used herein, synapse may be a synapse formed between a neuron and another neuron (e.g., a synapse formed between an axon of a neuron and a dendrite of another neuron) or a synapse formed between a neuron and another type of cell (e.g., a muscle cell), but is preferably a synapse formed by the presynapse of a neuron and the postsynapse on a skeletal muscle cell (also referred to as a "neuromuscular junction").

As used herein, "presynapse (presynaptic region)" refers to the ampullar portion in a synapse, which is formed at the axon terminal of a neuron, and "postsynapse (postsynaptic region)" refers to the portion facing the presynapse of another cell, such as the dendrite of another neuron or skeletal muscle, organ, or the like. In addition, the "synaptic cleft" refers to a space between the presynapse and postsynapse. In a synapse, signals are transmitted by binding to receptors located in the postsynapse by the release of neurotransmitters accumulated in a synaptic vesicle present in the presynapse into the synaptic cleft.

As used herein, "synaptic vesicle" refers to secretory vesicles present in the cytoplasm of the neuron for presynapse. Synaptic vesicle herein includes not only vesicles that contain a neurotransmitter therein and fuse with the cell membrane in response to a stimulus to release the neurotransmitter into synaptic cleft, but also vesicles that are recovered into the neurons by endocytosis (including bulk endocytosis) after release of the neurotransmitter.

The targeting agent according to the present invention binds to the intravesicular domain of a membrane protein, such as Synaptotagmin 2, exposed on the cell membrane in synaptic cleft, and is incorporated into synaptic vesicle by endocytosis so that it can be delivered to the cell body or the like of the motor neuron. Thus, with the targeting agent according to the present invention, the desired substance (label substance and/or physiologically active substance) can be targeted to the interior of motor neuron, in particular to the cell body of motor neuron.

As used herein, "antibody capable of binding to the intravesicular domain of a membrane protein (intravesicular domain antibody)" refers to an antibody recognizing the intravesicular domain of a membrane protein as an antigen and can specifically bind thereto.

For example, as used herein, "an antibody capable of binding to the intravesicular domain (N-terminal portion) of Synaptotagmin 2 (anti-SYT2 N-terminal antibody)" refers to an antibody recognizing the intravesicular domain of Synaptotagmin 2 as an antigen and can specifically bind thereto.

Anti-intravesicular domain antibody, such as an anti-SYT2 N-terminal antibody, includes both monoclonal antibody and polyclonal antibody, and an antibody included may also be an IgG antibody molecule, an IgM antibody molecule, or an antigen-binding fragment and an antigen-binding derivative thereof. For example, an antibody may be a complete antibody, Fab, Fab', F(ab')₂ fragment, or a single-chain antibody (scFv) fragment, in which the heavy chain variable region (VH) and the light chain variable region (VL) are linked via a linker, scFv-Fc, sc(Fv)₂, Fv, diabody, or the like. These antibodies can be easily obtained or synthesized by a person skilled in the art using the intravesicular domain of a membrane protein described above, such as the N-terminal polypeptide of Synaptotagmin 2, or intravesicular domain of other known membrane protein as an antigen. In addition, a commercially available product may be used as an antibody for the present invention. Antibody may be a human chimeric antibody, a humanized antibody or a human antibody, and where the conjugate or targeting agent according to the present invention is administered to a human, antibody moiety is preferably a human chimeric antibody, a humanized antibody or a human antibody.

The targeting agent according to the present invention may comprise antibodies capable of binding to multiple types of intravesicular domains. In this case, the multiple types of intravesicular domain may be of the same membrane protein or may be of differing membrane protein.

Antibody that can be used herein further includes a derivative that is understood by a person skilled in the art as long as it does not affect antigen-binding properties, such as a derivative with a modification for facilitating an antibody purification or enhancing the stability. As used herein, "antibody" is intended to include a fragment and a derivative that retains the binding capacity to the intravesicular domain of Synaptotagmin 2, unless otherwise specified by the context.

Instead of antibody herein, any molecule capable of binding to a molecule of interest can be used as the targeting agent or motor neuron visualizing agent according to the present invention. The molecular capable of binding to a molecule of interest includes, for example, an aptamer, a cyclic peptide, a receptor or a ligand of the molecule of interest, or a combination thereof.

The targeting agent according to the present invention may further comprise a desired substance (label substance and/or physiologically active substance) in addition to an antibody capable of binding to the intravesicular domain of a membrane protein, such as an antibody capable of binding to the intravesicular domain (N-terminal portion) of Synaptotagmin 2.

As used herein, "label substance" refers to an agent that emits a detectable signal indicating its presence. Label substance includes, for example, a luminescent label substance that emits light under a certain condition, such as a fluorescent molecule and a chemiluminescent substance, a sound-emitting label substance that emits sound waves, such as a photoacoustic effect probe, a radioactive label substance, and the like. Examples of fluorescent molecules include, but are not specifically limited to, a fluorescent molecule such as a fluorescent protein, fluorescein and a derivative thereof, pyrene and a derivative thereof, a quantum dot, and the like. The chemiluminescent substance includes, for example, an enzyme such as peroxidase (HRP) and alkaline phosphatase (ALP). The radioactive label substance includes, for example, a reagent including ¹⁴C, ³H, ¹²⁵I, and the like. The photoacoustic effect refers to a phenomenon in which a thermal elastic wave is generated due to adiabatic expansion caused by light absorption, and this thermal elastic wave can be detected as an acoustic wave. The photoacoustic effect probe includes, for example, indocyanine green or a derivative thereof, curcumin derivative, choline derivative, and the like. If the light absorption properties of the antibody, label substance, and physiologically active substance used are known, the label substance for the photoacoustic effect may not necessarily be used, and for example, the luminescent label substance may be detected on the basis of the photoacoustic effect.

As used herein, "physiologically active substance" refers to a substance capable of exerting a physiological effect directly or indirectly on an organism or a cell. The examples thereof include a low-molecular-weight compound, a functional middle molecule such as a peptide and an aptamer, and a polymeric compound including a biopolymer such as a protein such as an antibody and an enzyme, and a nucleic acid such as DNA and RNA, which is capable of exerting physiological effects on the target motor neuron. For example, a drug or prodrug such as a synaptogenesis-promoting agent, a synapse-maintaining agent, a muscle-enhancing agent or a neuronal function-modifying agent may be used as a physiologically active substance.

"Physiological effect" refers to an effect that results in a quantitative and/or qualitative change in a biomolecule such as a protein, DNA, or RNA. As a result of the physiological effect, for example, a function and a property of a living body, an organ, a tissue, a cell, and the like may be changed. For example, an effect such as promoting or suppressing synaptogenesis, improving or preventing an impairment in neural function, or improving or preventing an overactivity of neurons can be provided.

Where the targeting agent according to the present invention comprises a desired substance (label substance and/or physiologically active substance), the desired substance is comprised in a state of being not covalently linked (e.g., non-covalently linked) or covalently linked to the antibody capable of binding to the intravesicular domain of a membrane protein, such as the antibody capable of binding to the intravesicular domain of Synaptotagmin 2. When comprised in a state of being covalently linked, the desired substance and the antibody capable of binding to the intravesicular domain of a membrane protein, such as the antibody capable of binding to the intravesicular domain of Synaptotagmin 2, form a conjugate (referred to as "conjugate according to the present invention").

As used herein, "conjugate" refers to a substance in which two or more molecules are covalently linked. In particular, in the conjugate according to the present invention an antibody capable of binding to the intravesicular domain of a membrane protein and a desired substance (label substance and/or physiologically active substance) are linked. Specifically, for example, an antibody capable of binding to the intravesicular domain of Synaptotagmin 2 is linked to a desired substance (label substance and/or physiologically active substance).

The covalent and non-covalent bonds between the antibody and desired substance in the targeting agent according to the present invention are not particularly limited as long as it is a bond with which the antibody capable of binding to the intravesicular domain of Synaptotagmin 2 can arrive in the vicinity of the motor neuron in a state of being linked to the desired substance.

As used herein, "synaptogenesis-promoting agent" refers to a drug that has the function of promoting the formation of the presynapse and/or postsynapse. Synaptogenesis promotion includes, for example, enhancing the binding strength of the synapse: for example, (i) increasing the surface area and/or volume of the presynapse and/or postsynapse; (ii) increasing and/or qualitatively changing the amount, density, accumulation speed, accumulation frequency, or the like of proteins specifically expressed in the presynapse (e.g., Synapsin 1 or Synapsin 2, or the like); and (iii) increasing and/or qualitatively changing the amount, density, accumulation speed, accumulation frequency, or the like of proteins specifically expressed in the postsynapse (e.g., LRRTM family proteins).

As used herein, "synapse-maintaining agent" refers to a drug that has the function of suppressing the regression of the presynapse and/or postsynapse or assisting the function. Synapse maintenance includes, for example, suppressing the attenuation of the adhesion of synapse or assisting them: for example, (i) suppressing the decrease of the surface area and/or volume of presynapse and/or postsynapse or assisting them; (ii) suppressing the decrease and/or qualitative changes in the amount, density, accumulation speed, accumulation frequency, or the like of proteins specifically expressed in the presynapse (e.g., synapsin 1 or synapsin 2) or assisting them; and (iii) suppressing the decrease and/or qualitative changes in the amount, density, accumulation speed, accumulation frequency, or the like of proteins specifically expressed in the postsynapse (e.g., LRRTM family proteins) or assisting them.

As used herein, "muscle-enhancing agent" refers to a drug that has the function of suppressing the attenuation of or enhancing muscule, or promoting the function. The type of muscle enhancement is not particularly limited as long as the function of the muscle is strengthened and may include: for example, the function of increasing the surface area and/or volume of the muscle; the function of increasing and/or qualitatively changing the density, the number, or the like of each element constituting the muscle, such as the muscle bundle, the muscle fiber, the myofibrils, the sarcomere, the muscle cells, and the like and/or causing a change in the expression level of a specific protein in the cells constituting the muscle; the function of increasing the muscle mass and/or the muscle strength (for example, the muscle mass or the muscle strength of the skeletal muscle); or the function of causing an attenuation of the muscle with these functions.

Specific synaptogenesis-promoting agent, synapse-maintaining agent and muscle-enhancing agent include, but are not limited to, for example, the compounds disclosed in JP 2022-053535 A (e.g., thiamine and derivatives thereof) and the compounds disclosed in JP 2023-028848 A (e.g., atropine, busulfan, chromocarb, procaineamide, udenafil, propifenazone, and derivatives thereof) found by the present inventors.

As used herein, "neuronal function-modifying agent" refers to a drug having the function of altering or promoting a function exerted by neuron. Functional alteration of a neuron is not particularly limited as long as the degree and/or nature of the function of the neuron changes and includes, for example, changes in the electrophysiological properties of the neuron (such as the properties of conduction and transmission of stimuli), changes in the mode of gene expression, and changes in morphological properties (such as the elongation, regression, and branching of neurite, and the formation and regression of synapse).

Specific functional modifier includes, but are not limited to, for example, AP-1 inhibitor (such as compounds disclosed in WO2020/196725 found by the present inventors), FUS inhibitor, SOD1 inhibitor, TDP-43 inhibitor (e.g., compounds of anacardic acid and the like), KIF1A inhibitor, cytoskeleton-modifying agent such as monomethyl auristatin E (MMAE), and the like.

As used herein, "cytoskeleton-modifying agent" refers to a drug that suppresses and/or promotes one or more selected from the group consisting of formation, maintenance, degradation, branching, running, and localization of the cytoskeleton. The cytoskeleton-modifying agent in the present specification also includes an agent that modifies the formation of a cytoskeleton or the like by acting on a molecule other than the cytoskeleton. The cytoskeleton includes any of microtubules, intermediate filaments and actin filaments. For example, as the cytoskeleton-modifying agent, an agent that suppresses the formation and maintenance of the cytoskeleton, specifically, for example, an inhibitor of microtubule polymerization or the like, may be used.

These agents may have a function on a neuron and may not have a motor neuron-specific function.

In the conjugate according to the present invention, the intravesicular domain antibody, such as the anti-SYT2 N-terminal antibody, and the label substance and/or physiologically active substance may be directly covalently linked, or they may be indirectly linked via a linker or the like.

Where the targeting agent according to the present invention does not comprise the conjugate, it is preferred that the label substance and/or physiologically active substance have a portion capable of binding to the intravesicular domain antibody, such as the anti-SYT2 N-terminal antibody. Specifically, for example, the label substance and/or physiologically active substance covalently linked to the antibody capable of binding to the intravesicular domain antibody, such as the anti-SYT2 N-terminal antibody, may be used. The terms "antibody capable of binding to the anti-SYT2 N-terminal antibody" and "antibody capable of binding to the intravesicular domain antibody" herein are in accordance with the description of the definitions of "anti-SYT2 N-terminal antibody" and "intravesicular domain antibody", except that the antigen thereof is the anti-SYT2 N-terminal antibody.

The linkage between the moiety capable of binding to the intravesicular domain antibody, such as the anti-SYT2 N-terminal antibody, and the label substance and/or physiologically active substance is in accordance with the linkage in the targeting agent or conjugate according to the present invention. Thus, the moiety capable of binding to the intravesicular domain antibody, such as the anti-SYT2 N-terminal antibody, and the label substance and/or physiologically active substance may be non-covalently linked, directly covalently linked, or indirectly linked via a linker or the like.

Where the targeting agent according to the present invention comprises the label substance and/or physiologically active substance, the label substance and/or physiologically active substance may be comprised in the targeting agent according to the present invention in the form of a peptide complex in which the intravesicular domain antibody, such as the anti-SYT2 N-terminal antibody, is non-covalently linked to the label substance and/or physiologically active substance.

The site at which the label substance and/or physiologically active substance binds to the intravesicular domain antibody, such as the anti-SYT2 N-terminal antibody, is not particularly limited as long as it does not interfere with the binding of the intravesicular domain antibody to the antigen, such as the binding of the anti-SYT2 N-terminal antibody to the SYT2 N-terminus. Specifically, for example, the label substance and/or physiologically active substance may be bound to the constant region or a site other than the hypervariable region (HVR).

In addition, multiple types of label substance and/or physiologically active substance may be comprised as long as they do not interfere with each other's function. In this case, for example, multiple identical substances may be comprised, and one or multiple of each of the different substances may be comprised.

As the linker that may be used herein, a linker suitably used in the art may be used as appropriate. The structure and the chain length of the linker herein may be appropriately selected as long as they do not interfere with the function of the resulting conjugate. The linker may, for example, be configured to be cleavable after being transported to the synapse. The linker may also be configured, for example, not to be cleaved after being transported to the synapse.

The linker may be any one commonly used in the art, and is not particularly limited, but, for example, a peptide linker composed of 5 to 25, preferably 10 to 20 amino acid residues, such as a GS linker, may be suitably used. A cleavable linker, for example, an acid-labile linker, a photolabile linker, a peptidase-sensitive linker, a dimethyl linker, a disulfide-containing linker, or the like, may also be used.

Intravesicular domain antibody binds to its antigen, the intravesicular domain of a membrane protein, that is transiently exposed to the cell surface by fusion of synaptic vesicle with cell membrane, and can be delivered intracellularly along with the membrane protein via endocytosis the synaptic vesicle. The anti-SYT2 N-terminal antibody comprised in the targeting agent according to the present invention binds to the intravesicular domain of Synaptotagmin 2, that is transiently exposed to the cell surface by fusion of synaptic vesicle with cell membrane, and can be delivered intracellularly along with Synaptotagmin 2 via endocytosis of the synaptic vesicle. Thus, the targeting agent or conjugate is preferably designed to deliver the label substance and/or physiologically active substance to the synaptic vesicle of the target synapse. The characteristics of compounds that can be delivered to the synaptic vesicle are well known in the art. Since the diameter of the endosome of the bulk endocytosis is 90nm-160nm, the particle diameter of the targeting agent or conjugate according to the present invention may have a mean (or a median) of, for example, 160nm or less, 150nm or less, 140nm or less, 130nm or less, 120nm or less, 110nm or less, 100nm or less, or 90nm or less. Additionally, since the diameter of the synaptic vesicle is 40nm-60nm, the particle diameter of the conjugate according to the present invention may have a mean (or a median) of, for example, 60nm or less, 55nm or less, 50nm or less, 45nm or less, 40nm or less, 35nm or less, 30nm or less, 25nm or less, 23nm or less, 20nm or less, 18nm or less, 15nm or less, 14nm or less, 13nm or less, 12nm or less. For example, if the targeting agent itself does not comprise the label substance and/or physiologically active substance, the entire particle diameter when the label substance and/or physiologically active substance are bonded to the targeting agent may be within the above-described ranges. However, the particle diameter may be designed to be large for the purpose of, for example, inhibiting endocytosis of the synaptic vesicle or delivering a substance to the surface of the synapse or synaptic cleft. Further, for example, if the label substance and/or physiologically active substance are designed to be already separated when delivery to the synaptic vesicle is made, the particle diameter prior to separation may be designed to be large.

The conjugate or targeting agent according to the present invention may not be configured to cross the blood-brain barrier. Normally, the conjugate or targeting agent according to the present invention does not cross the blood-brain barrier and therefore does not act on the central nervous system and can only act on the peripheral synapse.

Targeting function to the motor neuron via synapse can be determined, for example, by administering the conjugate or targeting agent according to the present invention, comprising the physiologically active substance, to a subject such as a vertebrate (e.g., a non-human mammal, human, or other vertebrate) and assessing the physiological effects of the conjugate or targeting agent according to the present invention on the neuron of the subject. The physiological effect can be assessed, for example, by comparing the degree of physiological effect between the group receiving the conjugate or targeting agent according to the present invention and the group without receiving it, and/or by comparing the degree of physiological effect between the group receiving the conjugate or targeting agent according to the present invention and the group receiving the physiologically active substance alone.

The present inventors have previously found that presynapse formation can be induced by co-culturing a neuron with microbeads on the surface of which a LRRTM molecule (such as the extracellular domain of LRRTM2) is immobilized (WO2021/006075).

Thus, whether a tested substance has a targeting function on a motor neuron, including the synapse, can also be determined by examining whether the tested substance is localized on the presynapse induced by co-culturing of neurons and microbeads.

"LRRTM (leucine-rich repeat transmembrane neuronal protein) family protein" refers to proteins belonging to LRRTM family. LRRTM family is one of Synapse organizer protein families on the side of postsynapse and has the activity of inducing presynapse formation. In mammals, including humans, four types of LRRTM family proteins have been reported: LRRTM1, LRRTM2, LRRTM3, and LRRTM4. LRRTM family protein used for the microbeads may be any of them.

### < Conjugate >

The present invention relates to a conjugate of an antibody capable of binding to the intravesicular domain of a membrane protein present in a synaptic vesicle (referred to as "intravesicular domain antibody"), such as an antibody capable of binding to the intravesicular domain (N-terminal portion) of Synaptotagmin 2 (referred to as "anti-SYT2 N-terminal antibody"), and the label substance and/or physiologically active substance. The conjugate according to the present invention is, for example, delivered to a motor neuron by incorporation into a synaptic vesicle of the motor neuron.

### < Visualizing Agent of a Motor Neuron or Synapse thereof >

The present invention provides a targeting agent, which is a visualizing agent of a motor neuron or synapse thereof (referred to as a "visualizing agent according to the present invention").

The visualizing agent according to the present invention is a targeting agent comprising a label substance for use in visualizing a motor neuron or synapse thereof.

"Visualizing motor neuron" refers to making all or part of motor neuron in a detectable state. When visualizing a part of a motor neuron, the part to be visualized may be a random or predetermined part. For example, synapse can be visualized as a predetermined part. The visualizing agent according to the present invention can then be used as a synapse visualizing agent. "Visualizing synapse" refers to making presynapse and/or postsynapse in a detectable state. Therefore, any detectable label substance, as well as the label substance that can be directly detected by visual observation, may be used in the visualizing agent according to the present invention. Similarly, the visualizing agent according to the present invention can visualize an axon terminal, an axon, an axon hillock, a cell body, a dendrite, and the like of a motor neuron.

The visualizing agent according to the present invention may be used in vivo or in vitro. The signal of the label substance may be detected while the motor neuron is alive or after the motor neuron is fixed. Label substance suitable for detecting a motor neuron in a living condition are known in the art. For example, a fluorescent substance known in the field of in vivo imaging, a luminescent substance such as a chemical or bioluminescent substance, a sound-emitting substance such as a photoacoustic probe, a radioactive substance such as a radioisotope, a contrasting agent, and the like may be included.

The visualizing agent according to the present invention may be used for any application, for example, to visualize the number, size, or position of motor neurons, synapses (including neuromuscular junctions) or synaptic vesicles, or to visualize tissue in a surgical or diagnostic procedure.

The visualizing agent according to the present invention may be provided in the form of a kit together with, for example, other reagents, such as reagents required for detecting the label substance comprised in the visualizing agent according to the present invention.

In particular, if, for example, the label substance is an enzyme or the like, the substrate can be provided with the visualizing agent according to the present invention.

### < Composition or Pharmaceutical Composition >

The present invention further relates to a composition (a "composition" according to the present invention) or a pharmaceutical composition (a "pharmaceutical composition according to the present invention") comprising a conjugate or a targeting agent according to the present invention.

A composition or a pharmaceutical composition according to the present invention comprises the targeting agent according to the present invention comprising a physiologically active substance. In addition to the targeting agent, the composition or pharmaceutical composition according to the present invention may optionally comprise an additive (such as a carrier (a solid, liquid carrier, and the like), an excipient, a surfactant, a binder, a disintegrant, a lubricant, a solubilizing agent, a suspending agent, a coating agent, a colorant, a preservative, a buffer, a pH modifier), and the like. The additive hereby may be appropriately selected according to the formulation or dosage form of the composition or pharmaceutical composition.

The composition or pharmaceutical composition according to the present invention may be prepared in any dosage form, such as, but not limited to, a solid formulation, a liquid formulation, a gel, an aerosol, or the like. If the composition or pharmaceutical composition is used as a liquid formulation, it may also be prepared as a dry product intended for reconstitution with, for example, saline immediately prior to its use.

The excipient includes, for example, lactose, crystalline cellulose, starch, and the like. The binder includes, for example, starch paste, gum arabic paste, hydroxypropylcellulose, and the like. The disintegrant includes, for example, starch, celluloses, carbonates, and the like. The lubricant includes, for example, wax, talc, and the like.

When the composition or pharmaceutical composition according to the present invention comprising the synaptogenesis-promoting agent, the synapse-maintaining agent, or the muscle-enhancing agent as a physiologically active substance, the impairment in neural function can be improved or prevented by promoting synaptogenesis. Therefore, the composition or pharmaceutical composition according to the present invention can be used to ameliorate or prevent an impaired neural function, for example, an impairment in neural function due to nerve injury, an impairment in neural function due to aging, an impairment in neural function due to diseases or the like, or to improve neural function.

As used herein, "nerve damage" refers to damage at any point in the nerve, including damage physically caused from outside the body and damage caused by factors in the body such as cancer, tumors, and the like.

As used herein, "aging" refers to various functional impairments, changes in morphology, changes in appearance, and the like that occur in an individual of an organism over time, and the processes thereof.

Frail, sarcopenia, and the like are known as conditions caused by aging. "Frail" refers to a condition in which mental and physical vitality (motor function, cognitive function, or the like) are reduced with aging, impairments in living functions, and mental and physical weakness occur, while being affected by coexistence of multiple chronic diseases, or the like. The reduction in mental and physical vitality includes, for example, cognitive dysfunction, dizziness, eating disorders, dysphagia, visual impairment, depression, anemia, hearing loss, delirium, compromised infectivity, weight loss, muscle mass loss, and the like. The chronic disease includes hypertension, heart disease, cerebrovascular disease, diabetes, respiratory disease, malignancy, and the like. On the other hand, sarcopenia means a condition in which muscle strength is reduced while skeletal muscle mass is reduced due to aging, disease, or the like. In the neuromuscular junction of aged mice, morphological changes such as synaptic detachment and partial or complete axonal detachment from the postsynapse are observed, and thus, it is believed that changes in the morphology of the neuromuscular junction with aging are involved in the reduction of skeletal muscle mass and the like in sarcopenia.

The composition or pharmaceutical composition according to the present invention can be used to ameliorate or prevent an impairment in neural function due to aging, particularly in a subject having or at increased risk of having frail or sarcopenia.

In the present invention, disease includes, for example, neurological disease and neuromuscular disease.

As used herein, "neurological disease" refers to a disease caused by a disorder of a nerve, such as a central nerve or a peripheral nerve, and refers to, for example, one or more diseases selected from the group consisting of Alzheimer's disease, Parkinson's disease, dementia of Lewy bodies, frontotemporal lobar degeneration, progressive supranuclear palsy, corticobasal degeneration, Huntington's disease, dystonia, prion disease, chorea-acanthocytosis, adrenoleukodystrophy, multiple system atrophy, spinocerebellar degeneration, amyotrophic lateral sclerosis, primary lateral sclerosis, bulbar spinal muscular atrophy, spastic paraplegia, syringomyelia, Charcot-Mary Tooth disease, frontotemporal dementia, epilepsy, schizophrenia, autism, autism spectrum disorder, or the like.

As used herein, "neuromuscular disease" refers to a disease caused by a disorder of any of the motor nerves, neuromuscular junctions, or muscle cells, for example, one or more diseases selected from the group consisting of muscular dystrophy, myopathy, congenital myasthenia syndrome, hereditary periodic quadriplegia, myasthenia gravis, Lambert-Eaton syndrome, and the like.

Amyotrophic lateral sclerosis (ALS) is a disease in which the primary motor nerve and the secondary motor nerve are selectively and progressively degenerated and disappear, and it is known that the motor nerve is detached from the skeletal muscle at the neuromuscular junction as an initial pathology. Thus, the composition or pharmaceutical composition according to the present invention comprising the synaptogenesis-promoting agent or synapse-maintaining agent capable of promoting the formation of synapse between skeletal muscle and motor nerve or suppressing the regression can be used to ameliorate or prevent, in particular, an impairment in neural function due to amyotrophic lateral sclerosis.

For example, the pharmaceutical composition according to the present invention may be a pharmaceutical composition for the treatment of amyotrophic lateral sclerosis, a pharmaceutical composition for the treatment of spinal muscular atrophy, or the like, comprising the conjugate or targeting agent according to the present invention.

A composition or pharmaceutical composition according to the present invention comprising the muscle-enhancing agent can improve or prevent muscle weakness by augmenting muscle. Thus, the composition or pharmaceutical composition according to the present invention comprising the muscle-enhancing agent can be used to ameliorate or prevent muscle weakness, such as muscle weakness following trauma or surgery, muscle weakness due to aging, muscle weakness due to diseases, or to improve muscle function.

As used herein, "trauma" refers to tissue or organ damage due to external factors, including, for example, wounds, fractures, sprains, visceral ruptures, burns, frostbites, and the like.

The composition or pharmaceutical composition according to the present invention comprising the function-modifying agent may improve or prevent an impairment or an increase in neurons by altering the function of a motor neuron. Thus, the composition or pharmaceutical composition according to the present invention comprising the function-modifying agent can also be used to ameliorate or prevent neuronal overactivity, such as neuronal overactivity due to a disease or condition, or to ameliorate or prevent muscle tone, such as muscle tremor due to aging or muscle tone due to trauma or disease.

Specifically, it can be used to improve or prevent various diseases and conditions described above, including abnormal involuntary movements (dyskinesias) (e.g., abnormal head movements, tremors, (painful) convulsions, muscle fasciculations), abnormal gait and movement (e.g., atactic gait, difficulty walking), other coordination disorders (e.g., ataxia), other conditions related to the nervous system and musculoskeletal system (e.g., tetany, abnormal reflexes, postural abnormalities, spasticity, muscle hypertonia, muscle tone, exaggerated deep tendon reflexes, dysphagia), and the like.

As used herein, "disease" refers to a pathological condition that can be classified by identifiable symptoms or causes in a subject individual and includes illness and disorders. In the present invention, "condition" refers to a pathological condition including a discernible symptom in a subject individual that is not encompassed in a disease.

The composition or pharmaceutical composition according to the present invention may comprise multiple targeting agent according to the present invention and may further contain another active ingredient. The active ingredient is not particularly limited as long as it does not interfere with the function of the targeting agent comprised in the composition or pharmaceutical composition. When multiple types of targeting agents are comprised, the membrane protein to which each targeting agent is capable of binding may be the same or different. Additionally, for example, the targeting agents comprising the antibody capable of binding to distinct sites in the same membrane protein may be comprised. The label substance and/or physiologically active substance comprised in these targeting agents may be different or the same from each other.

### < Method for Targeting to a Motor Neuron or Synapse >

The present invention relates to a method for targeting to a motor neuron or synapse. According to the present invention, targeting to a motor neuron (e.g., an axon terminal, an axon, an axon hillock, a cell body, an dendrite, and the like of motor neuron) is achieved by contacting a motor neuron with an antibody capable of binding to the intravesicular domain of a membrane protein present in the synaptic vesicle (referred to as "intravesicular domain antibody"), such as the antibody binding to the intravesicular domain of Synaptotagmin 2 (anti-SYT2 N-terminal antibody), and allowing the antibody to be incorporated into motor neuron (within synaptic vesicle of motor neuron, in particular). In this way, the intravesicular domain antibody, including the antibody binding to the intravesicular domain of Synaptotagmin 2, is targeted to motor neuron or synapse. The desired substance can be delivered within a motor neuron (e.g., within a synaptic vesicle of the motor neuron) by directly or indirectly linking the desired substance (e.g., label substance and/or physiologically active substance) to the antibody via a linker to produce the conjugate according to the present invention and contacting the conjugate with the motor neuron, or by separately contacting the antibody and desired substance capable of binding to the antibody with the motor neuron. Thus, according to the present invention, there is provided a method for targeting to a motor neuron or synapse comprising contacting cells, such as the motor neuron, with an antibody (which may be linked to a desired substance).

### < Method for Targeting the Label Substance and/or Physiologically Active Substance >

The present invention relates to a method for targeting a label substance and/or a physiologically active substance comprising a step of contacting a conjugate or a targeting agent according to the present invention with a motor neuron and a step of delivering the targeting agent to the motor neuron synapse.

The subject in the present invention is not particularly limited as long as it comprises a motor neuron. The contact may be performed using, for example, a motor neuron alone as a subject or a tissue comprising cells other than the motor neuron as a subject. For example, because the targeting agent according to the present invention primarily targets the presynaptic region of motor neuron synapse at the neuromuscular junction, it may be directed to a tissue further comprising skeletal muscle cells projected by motor neuron.

Motor neuron in the present method may comprise motor neuron of a vertebrate (non-human mammal, human, or other vertebrate). Motor neuron in the present method preferably comprises human motor neuron. Hereinafter, the present invention will be described with reference to human motor neuron, but the present method is not limited to that of human motor neuron.

The human motor neuron may be used without limitation regardless of its origin. Examples include, but are not limited to, a primary culture of a cell isolated from humans, a cell isolated from humans and established as a cell line, and a human motor neuron into which a pluripotent stem cell derived from humans is induced to differentiate. The pluripotent stem cell from which the human motor neuron is derived is preferably a pluripotent stem cell from a human.

As used herein, the term "pluripotent stem cell" refers to a cell that is capable of self-renewal, can be cultured in vitro, and has pluripotency capable of differentiating into cells constituting an individual. Specific examples thereof include an embryonic stem cell (ES cell), a pluripotent stem cell derived from a fetal primordial germ cell (GS cell), an induced pluripotent stem cell derived from a somatic cell (iPS cell), and the like, but a human-derived iPS cell or ES cell is preferably used in the present method.

The ES cell is often obtained from a fertilized egg but can also be obtained from other than a fertilized egg, such as adipose tissue, placenta, a testicular cell, and any ES cell is the subject of the present invention. The methods for producing an ES cell from other than a fertilized egg have been reported (e.g., WO2003/046141), and these reports can be referred to and used as appropriate.

The iPS cell is an artificial stem cell derived from somatic cells, can be produced by introducing specific reprogramming factors into a somatic cell in the form of nucleic acids or proteins, and exhibits properties almost comparable to those of the ES cell (e.g., pluripotency and proliferative capacity based on self-renewal). Examples of genes encompassed in the reprogramming factors include Oct3/4, Sox2, Sox1, Sox3, Sox15, Sox17, Klf4, Klf2, c-MYC, N-Myc, L-Myc, Nanog, Lin28, Fbx15, ERas, ECAT15-2, Tcl1, beta-catenin, Lin28b, Sall1, Sall4, Esrrb, Nr5a2, Tbx3, Glis1, or a combination thereof. Many reports for methods for producing an iPS cell are provided, and these reports may be referred to, and may be appropriately modified and used. The iPS cell that can be used in the present method is preferably a human-derived iPS cell, for example, a human fibroblast-derived iPS cell.

Many reports for methods for inducing differentiation of human iPS cells into peripheral neurons have been provided (e.g., Chambers, Stuart M., et al., Nature biotechnology 27.3 (2009): 275.), and these reports may be referred to, and may be appropriately modified and used. Alternatively, various neural cells into which a human iPS cell is induced to differentiate are commercially available and can be purchased, for example, from FUJIFILM Cellular Dynamics, Inc., Reprocell Inc., or the like.

In one embodiment, the present method is a method of in vitro. In another embodiment, the present method is a method of ex vivo. In another embodiment, the present method is a method of in vivo. Where the present method is a method of in vivo, the subject may be a mammal other than a human.

Where the present method is a method of in vitro, the present method may further comprise the step of inducing synaptogenesis. As used herein, "inducing synaptogenesis" refers to causing the formation of the presynapse in the axon of a neuron and/or causing the formation of the postsynapse at a dendrite of another neuron, or at a cell such as of skeletal muscles or organs. Synaptogenesis may be induced by co-culturing a cell to form presynapse and a cell to form postsynapse. Synaptogenesis may also be induced by other methods, for example, by co-culturing a motor neuron with beads coated with the extracellular domain of LRRTM2. The step of inducing synaptogenesis may be performed simultaneously with or prior to the step of contacting the targeting agent according to the present invention with a motor neuron.

Where the present method is a method of in vitro, the step of contacting the targeting agent according to the present invention with a motor neuron is performed by contacting the targeting agent according to the present invention with a sample containing a motor neuron. The method of contacting is not particularly limited as long as a motor neuron in the sample and targeting agent may be brought into contact with each other. For example, targeting agent may be applied to the sample by directly sprinkling, spraying, dropping, spreading, immersing the sample in targeting agent, or a combination thereof. In addition, where the sample is present in another carrier (for example, a culture medium or the like), the targeting agent may be applied to the carrier by sprinkling, spraying, dropping, or spreading.

The amount to be applied is not particularly limited and may be appropriately set considering the number of motor neurons and other conditions. The application may be performed at a concentration of, for example, 0.01µg/mL or more, 0.1µg/mL or more, 0.2µg/mL or more, 0.5µg/mL or more, 0.7µg/mL or more, 0.9µg/mL or more, 1µg/mL or more, 2µg/mL or more, 5µg/mL or more, 7µg/mL or more, 9µg/mL or more, or 10µg/mL or more, as a concentration of the IgG antibody.

Where the present method is a method of in vivo, the step of contacting the targeting agent according to the present invention with the test sample is performed by administering the targeting agent according to the present invention to the subject.

Examples of the administration method include, but are not particularly limited to, topical administration, enteral administration, and parenteral administration, specifically, dermal administration, inhalation administration, enema administration, eye drop, ear drop, nasal administration, intravaginal administration, tube feeding, intravenous administration, intraarterial administration, intramuscular administration, intracardiac administration, subcutaneous administration, intraosseous administration, intradermal administration, intrathecal administration, intravesical administration, transdermal administration, transmucosal administration, epidural administration, intravitreal administration, and the like.

The dose is not particularly limited and may be appropriately set considering the animal species of the subject and other conditions. For example, where IgG antibody is administered to mice, the dose per 1kg of body weight may be 0.1mg/kg or more, 0.5mg/kg or more, 1mg/kg or more, 2mg/kg or more, 4mg/kg or more, or 5mg/kg or more.

The targeting agent used in this step may not be of one type. For example, multiple types of targeting agents may be used together or separately. Specifically, for example, the targeting agent comprising a conjugate and the targeting agent not comprising a label substance and/or a physiologically active substance may be used. Additionally, a label substance and/or physiologically active substance used may not be one type, and for example, multiple types of label substance and/or physiologically active substance may be used together or separately. Specifically, for example, a label substance and a physiologically active substance may be used in combination.

The step of contacting the targeting agent according to the present invention with a motor neuron may be performed multiple times. Where this step is performed multiple times, the types of the targeting agent and cell, as well as the method of application and administration used in each performance, may be the same or different each time.

Upon contacting the motor neuron with the conjugate or targeting agent according to the present invention, the targeting agent according to the present invention is incorporated into the motor nerve via synaptic vesicle. When the targeting agent according to the present invention is contacted with the motor neuron, the motor neuron may be activated, or the activity thereof may be promoted. By activating or promoting the activity of the motor neuron, the conjugate or targeting agent according to the present invention can be more efficiently incorporated into the motor neuron. Usually, when the conjugate or targeting agent according to the present invention is incorporated into a synaptic vesicle, it is delivered to the cell body through axons by retrograde transport.

The methods of activating a motor neuron include, but are not particularly limited to, for example, the method to allow spontaneous activity of the motor neuron over a sufficient period of time and the method of activating motor neuron or promoting endocytosis of synaptic vesicle by artificial stimuli.

Methods to allow spontaneous activity include, for example, placing a motor neuron under the environment to allow activity over a sufficient period of time. The environments to allow a motor neuron to be active are well known in the art.

Time period for activity in the method to allow spontaneous activity of a motor neuron (e.g., the time period of contacting with the conjugate or targeting agent according to the present invention) is not particularly limited but may be, for example, where the present method is an in vitro method, 1 hour or more, 3 hours or more, 6 hours or more, 12 hours or more, 18 hours or more, or 24 hours or more. Where the present method is an in vivo method, it may be, for example, 1 hour or more, 3 hours or more, 6 hours or more, 12 hours or more, 18 hours or more, 24 hours or more, 36 hours or more, 48 hours or more, 60 hours or more, 72 hours or more, 100 hours or more, 120 hours or more, 150 hours or more, 168 hours or more, 200 hours or more, or 240 hours or more.

Methods of activating or promoting the activity of a motor neuron by artificial stimuli include, for example, placing the motor neuron under an environment where a motor neuron can be highly active for a sufficient period of time.

Where the present method is an in vitro method, a chemical stimulus and/or a physical stimulus can be provided to the motor neuron in the method of activating or promoting the activity of the motor neuron. Stimuli for highly activating a motor neuron are well known in the art. Compounds used for chemical stimulation include, for example, a potassium ion channel inhibitor such as amiodarone, tetraethylammonium, 4-aminopyridine, barium, dendrotoxin, a sodium channel agonist such as batracotoxin, a calcium channel agonist such as Bay K8644, high concentration of potassium ions or neurotransmitters, or combinations thereof. Examples of the physical stimulus include a temperature change.

When chemical stimulation is performed, the added amount of the compound is not particularly limited. For example, addition may be at a concentration of 1µM or more, 10µM or more, 50µM or more, or 100µM or more.

The duration of the stimulation is not particularly limited and can be appropriately set considering conditions such as the type and intensity of the stimulation. For example, stimulus may be given for 2 minutes or more, 3 minutes or more, 4 minutes or more, 5 minutes or more, 8 minutes or more, 9 minutes or more, 10 minutes or more, 20 minutes or more, 25 minutes or more, 30 minutes or more, or 1 hour or more.

Where the present method is an in vivo method, the method of activating or promoting the activity of a motor neuron may be performed by, such as, a method to allow the subject to be highly active (e.g., a method to allow the subject to have exercise or a method of activating the brain activity), a method of promoting the activity of the motor neuron by a chemical or the like. Compounds that promote motor nerve activity include those compounds used for the chemical stimulation described above and may be administered to a subject. The compound used for the chemical stimulus may be administered at a pharmaceutically acceptable concentration or method. The compound may be administered to a subject such that the compound stimulus does not exert biotoxicity, but the compound may not be administered to the subject if the biotoxicity is manifested. Promoting motor neuron activity is usually expected to provide comparable effect in a short time compared to the method to allow firing naturally.

Where the targeting agent without a label substance and/or a physiologically active substance is used, the label substance and/or physiologically active substance and targeting agent may be contacted with a motor neuron together or separately. If contacting the label substance and/or physiologically active substance and the targeting agent separately, the timing is not particularly limited as long as the label substance and/or physiologically active substance can be combined with the targeting agent. For example, the contacting of the label substance and/or physiologically active substance may be started before the contacting of the targeting agent or after the contacting of the targeting agent.

The method for each contact may be selected as the method of contacting described above. For example, the same method or different methods may be used for each contact.

The present method may optionally further comprise the step of confirming success or failure of targeting. If the targeting agent used in the present method comprises a physiologically active substance, targeting can be determined to be successful if, for example, a physiological effect was observed as described above in the targeting agent section. In addition, if the targeting agent used in the present method comprises a label substance, targeting can be determined to be successful when the signal is detected, as in the step of detecting the signal of the label substance in the method for visualizing, which will be described later.

When the pharmaceutical composition according to the present invention is used as a targeting agent for the present method, the present method can be used as a method for preventing or treating a condition or a disease.

The present invention thereby relates to a method for preventing or treating a condition or a disease comprising a step of contacting a targeting agent comprising an antibody capable of binding to the intravesicular domain of a membrane protein present on the synaptic vesicle of a motor neuron and physiologically active substance with a motor neuron, and a step of delivering said targeting agent to said motor neuron synapse. According to the present method, various conditions or diseases exemplified with respect to the pharmaceutical composition can be prevented or treated. The contacting step of the present method preferably comprises administering the targeting agent and/or pharmaceutical composition to the subject.

Thus, for example, the method according to the present invention is a method for ameliorating or preventing an impairment in neural function, such as an impairment in neural function due to nerve damage, an impairment in neural function due to aging, or an impairment in neural function due to a disease, or for improving neural function. In one embodiment, the condition or disease is a condition or a disease that exhibits impaired neural function. In one embodiment, the condition or disease is a neurological disease and a neuromuscular disease. In one embodiment, the targeting agent comprises a conjugate of the antibody and the physiologically active substance.

In this case, the method may further comprise a step of sufficiently exerting a physiological effect in the subject. For example, when a physiologically active substance to be used is a substance capable of exerting an effect alone, the effect can be exerted by placing the subject in an environment in which nutrients are sufficiently provided for a sufficient period of time for the physiological effect to be exerted. Additionally, for example, if a physiologically active substance to be used requires another substance to exert an effect, the substance can be additionally administered.

The duration of this step may be determined as appropriate depending on the condition of the subject, the type of the physiologically active substance, the dose, and the like. For example, a determination may be made based on the duration generally required for a physiological effect to be exerted when the physiologically active substance is administered, and the physiological effect may be confirmed once or multiple times and continued until the physiological effect is sufficiently exerted.

### < Method for Visualizing a Motor Neuron or Synapse thereof >

The present invention relates to a method for visualizing a motor neuron or synapse comprising a step of contacting a visualizing agent according to the present invention with a motor neuron, a step of delivering the visualizing agent to a motor neuron synapse, and a step of detecting the signal of the label substance.

In the present method, the motor neuron used for contacting is as described for the method for targeting a label substance and/or a physiologically active substance described above.

In one embodiment, the present method is a method of in vitro. In another embodiment, the present method is a method of ex vivo. In another embodiment, the present method is a method of in vivo. Where the present method is an in vivo method, the subject may be a human or a mammal other than a human.

In the present method, the step of contacting the visualizing agent according to the present invention with a motor neuron and the step of delivering the visualizing agent to a motor neuron synapse are the same as the step of contacting the targeting agent according to the present invention with a motor neuron and the step of delivering the targeting agent to a motor neuron synapse described for the method for targeting a label substance and/or a physiologically active substance described above, except for using a visualizing agent as the targeting agent.

The present method may optionally further comprise a step of generating a signal from the label substance. The method of generating the signal is not particularly limited. The method of generating the signal and the necessity thereof may be determined based on the type of label substance to be used or the like.

For example, if the label substance is a fluorescent molecule or a radioactive label substance, a signal can be generated at the target by waiting for a time sufficient for the visualizing agent to be delivered to the target motor neuron synapse, that is, a time sufficient for the visualizing agent to reach the target motor neuron synapse and for endocytosis of a synaptic vesicle to occur at that motor neuron synapse. This time may be appropriately selected as in the time of the step of delivering a visualizing agent to a motor neuron synapse.

For example, if a label substance is a chemiluminescent material, it can be done by adding a substance, such as its substrate used to generate the signal, in addition to waiting for sufficient time for the visualizing agent to be delivered to the target motor neuron synapse.

This step may be performed at the same time as or before the step of detecting a signal described below.

The present method further comprises the step of detecting the signal of the agent for detection. The method used for detecting is not particularly limited and may be appropriately selected according to conditions such as the type of the label substance to be used.

Where the label substance is a fluorescent material, for example, a motor neuron may be irradiated with excitation light containing light with an excitation wavelength for the label substance, and the detection may be performed using a detector capable of detecting the fluorescence wavelength of the label substance. In addition, where the label substance is a chemiluminescent material, for example, the detection may be performed using a detector capable of detecting the emission wavelength of the label substance. Where the label substance is a radioactive label substance, the detection may be performed using a detector capable of detecting the radiation emitted by the label substance.

Detecting the signal of the label substance includes detecting the presence, location, or quantity of motor neuron (e.g., synapse, cell body, or the like) in a sample containing a motor neuron.

In addition to the step of detecting the signal of the label substance in the sample, the methods according to the present invention may further comprise the step of comparing the signal of the label substance detected in the sample to a signal in a standard sample comprising the label substance or a pre-established reference value to determine the presence, location or quantity. The standard sample is not particularly limited as long as it is a biological sample that serves as a reference for determining whether to have a specific condition or disease. Specifically, for example, those obtained from a healthy individual, those obtained from the same individual as the sample at different times of collection, or those obtained from an individual known to have a specific condition or disease. The standard sample may be, for example, a biological sample derived from the same biological species, individual, tissue or cell as the sample, or may be a biological sample derived from another biological species, individual, tissue or cell. Further, the reference value is not particularly limited as long as it is a reference value for determining whether to have a condition of interest. The reference value may be set based on, for example, the intensity, number, or the like of signals generally detected in the standard sample.

In any case, the method of comparison is not particularly limited. For example, comparison may be performed by visual observation, by numerical magnitudes, or by a statistical method.

### < Other Inventions >

The present invention provides a method of administering a substance to a subject. The substance is in the form of a conjugate of an intravesicular domain antibody, such as an anti-SYT2 N-terminal antibody, and with the substance. The substance can be thereby delivered to the cell, such as a motor neuron, of the subject. Where the substance is a physiologically active substance, the physiologically active substance can be delivered to a cell such as a motor neuron. Additionally, where the substance is a label substance, it can be used to observe the delivery site of the label substance (e.g., motor neuron or synapse thereof). The present invention also provides a conjugate of the antibody and the substance, or a composition comprising the conjugate for use in this method. The present invention relates to a composition for targeting to a motor neuron or a synaptic thereof comprising an antibody capable of binding to the intravesicular domain of a membrane protein present on the synaptic vesicle of a motor neuron, or a conjugate of the antibody with a label substance and/or a physiologically active substance.

The present invention provides a method for visualizing a motor neuron in a subject, comprising administering to the subject an effective amount of a conjugate of an intravesicular domain antibody, such as an anti-SYT2 N-terminal antibody, and a label substance. The present invention also provides a conjugate of the antibody and the label substance or a composition comprising the conjugate for use in this method.

The present invention provides a method of delivering a physiologically active substance to a motor neuron of a subject, comprising administering to the subject an effective amount of a conjugate of an intravesicular domain antibody, such as an anti-SYT2 N-terminal antibody and the physiologically active substance. The present invention also provides a conjugate of the antibody and the physiologically active substance or a composition comprising the conjugate for use in this method.

The present invention provides an intravesicular domain antibody or a conjugate of the intravesicular domain antibody and a label substance and/or a physiologically active substance for use in any of the above methods. For example, the present invention relates to an intravesicular domain antibody or a conjugate of the intravesicular domain antibody and the physiologically active substance for use in a method of preventing or treating a condition or a disease. Further, for example, the present invention relates to an intravesicular domain antibody or a conjugate of the intravesicular domain antibody and a label substance and/or a physiologically active substance for use in the method for targeting the label substance and/or the physiologically active substance to a motor neuron or a synapse thereof.

The present invention provides the antibody or a conjugate of the antibody and the substance for use in the manufacture of a medicament for use in any of the methods described above.

The present invention also provides the use of the intravesicular domain antibody or a conjugate of the intravesicular domain antibody and a physiologically active substance in the manufacture of a medicament comprising the antibody and the physiologically active substance. Examples

Hereinafter, the present invention will be described in more detail with reference to Example. However, the technical scope of the present invention is not limited to these Examples.

### < Example 1: Delivery of Synaptotagmin 2 antibody to a Human Motor Neuron Synapse >

Microbeads coated with the extracellular domain of LRRTM2 were used to determine whether a Synaptotagmin 2 antibody was delivered to a human motor neuron synapse and the condition therefor.

### 1. Cell Culture

Human iPS-derived motor neuron (40HU-005-2M; ixcells biotechnologies) was thawed using Dead Cell Removal kit (Veritas) according to the protocol of the kit. The cells after thawing were plated on 96-well plates (V-bottom) at a density of 2×10⁴ cells/well and cultured in the motor neuron culture medium (Motor Neuron Maintenance Medium; ixcells biotechnologies) for 1 week to prepare neurospheres. The prepared neurospheres were selected based on size and roundness, and those satisfying this criterion were used in the following experiments.

After coating 96-well EZVIEW (registered trademark) culture plate LB (AGC Technoglass Co., Ltd.) with poly-D-lysine and Geltrex (registered trademark) Matrix (Thermo Fisher Scientific), the selected neurospheres were plated and cultured for 20 days. As the medium, the above-described motor neuron culture medium was used first, and medium change with the same medium was performed on the second day of culture. Thereafter, medium was changed three times per week using neuronal medium (Neurobasal plus medium (B27 plus supplement (Thermo Fisher Scientific), 20ng/mL BDNF, 20ng/mL GDNF, and penicillin-streptomycin were added). All medium changes were performed at 50 µL/well.

### 2. Preparation of LRRTM2 Bead

Microbeads coated with the extracellular domain of LRRTM2 (active beads) were prepared as disclosed in WO2021/006075.

Specifically, streptavidin-coated microbeads (Streptavidin Coated Microspheres; Bangs Laboratories, Inc.; polystyrene, mean diameter 9.94µm) were washed twice with wash buffer (phosphate buffered saline (PBS), 0.01% bovine serum albumin (BSA), 0.05% TritonX-100) and reacted with biotinylated anti-human IgG (Fc-specific) antibody (Sigma-Aldrich; mouse monoclonal) in binding buffer (PBS, 0.01% BSA) to immobilize the biotinylated anti-human IgG (Fc-specific) antibody to streptavidin-coated microbeads. The resulting beads were washed three times with wash buffer (anti-human IgG-Fc antibody beads).

Anti-human IgG-Fc antibody beads were then suspended in a binding buffer to which a fusion protein of the extracellular domain of human LRRTM2 and the Fc part of human IgG (LRRTM2-Fc; R&D systems) were added to immobilize LRRTM2-Fc on the anti-human IgG-Fc antibody beads. The resulting beads were washed with wash buffer and suspended in binding buffer (LRRTM2 bead suspension).

### 3. Induction of Presynapse (Figure 1)

Plates that had been cultured for 20 days were seeded with LRRTM2 beads at 0.1µg/well and cultured for 48 hours at 37°C to induce presynapse formation.

### 4. Preparation of Antibody Solution

As an antibody, a rabbit anti-SYT2 N-terminal antibody (Polyclonal rabbit purified antibody SYT2 lumenal domain; Cat. No. 105 223; Synaptic Systems), which can bind to a peptide having an amino acid sequence of SEQ ID NO: 5, or a normal rabbit IgG antibody (Normal Rabbit IgG; Cat. No. AB-105-C; R&D Systems), as a control, was used.

After the neuronal medium was warmed at 37°C for 30 minutes, the antibody was added to the medium to have a final concentration of 1µg/mL or 10µg/mL and mixed. The crude antibody solution was centrifuged at 200g for 3 minutes at room temperature, and the supernatant was collected as an antibody solution.

### 5. Introduction of an Antibody

Antibody was introduced by changing the culture medium with 100µL of antibody solution on the plate on which presynapse is formed.

### 6. Spontaneous Activity of Motor Neurons

After introduction of the antibody, spontaneous activity of motor neurons was facilitated by culturing at 37°C for 24 hours.

### 7. Fixation of Cells

After culturing, the cells were washed with neuronal medium and then fixed in 2% paraformaldehyde (PFA).

### 8. Immunocytochemical Staining

Immunocytochemical staining was performed using the added antibody as the primary antibody. The cells after fixation were permeabilized with detergent and blocked, followed by a primary antibody reaction using a mouse anti-βIII-tubulin (Tuj1) antibody (Cat. No. 801202; Biolegend) as an additional primary antibody. A secondary antibody reaction against each primary antibody was then performed to obtain fluorescent images. Blocking was performed using blocking buffer (PBS + 2% normal goat serum + 1% BSA + 1% fetal bovine serum + 0.02% TritonX-100).

The secondary antibodies used were as follows:
Alexa 488 labeled anti-rabbit antibody (Cat. No. A32731; Thermo Fisher Scientific);
Alexa 555 labeled anti-mouse antibody (Cat. No. A32727; Thermo Fisher Scientific).

Fluorescent images were obtained using an inverted live cell (DMi8) microscopic fluorescent microscope (Leica) equipped with LAS X software (Leica) at the following excitation wavelengths, detection wavelengths, exposure times, and detection thresholds. All gamma correction values were 1.

Excitation wavelength and detection wavelength:
Alexa 488 maximum excitation 490nm; maximum detection 525nm; actual detection 512nm;
Alexa 555 maximum excitation 555nm; maximum detection 580nm; actual detection 595nm.

Exposure time and detection threshold:
Alexa 488 exposure time 200ms; detection threshold 150-1500;
Alexa 555 exposure time 50ms; detection threshold 100-3500.

The results are shown in Figures 2-1 and 3. Figures 2-1 and 3 show immunocytochemical staining images for an antibody concentration of 1µg/mL and 10µg/mL, respectively.

In control, in which a normal rabbit IgG antibody was used as an antibody, no signal was observed under conditions with any concentration (Figure 2-1A and Figure 3A). On the other hand, where an anti-SYT2 N-terminal antibody was used as an antibody, a strong signal was observed on LRRTM2 beads under conditions with any concentration (Figure 2-1B and Figure 3B). On the LRRTM2 bead, postsynapse is absent and only the motor neuron presynapse is present. Accordingly, it was found that by targeting the N terminus of SYT2, an antibody is delivered to the surface of the LRRTM2 bead. In addition, since sufficient time has elapsed for endocytosis of synaptic vesicles, it is considered that the introduced anti-SYT2 N-terminal antibody is incorporated into motor neuron synaptic vesicles.

Further, Figure 2-2 shows magnified images of LRRTM2 beads in the experiment in which an anti-SYT2 N-terminal antibody was administered at a concentration of 1µg/mL. On LRRTM2 beads where the neuronal axons indicated by Tuj1 are not densely located (Figure 2-2A), neuronal axon terminals (points with an intense signal of Tuj1 in the figure) are not observed on the surface of LRRTM2 beads. Accordingly, it is considered that synapse is not formed on the surface of these LRRTM2 beads. On the other hand, on LRRTM2 beads where the neuronal axons are densely located (Figure 2-2B), neuronal axon terminals are observed on the surface of LRRTM2 beads. Accordingly, it is considered that synapse is formed on the surface of these LRRTM2 beads. Since no signal of anti-SYT2 N-terminal antibody was detected on the surface of the LRRTM2 bead in Figure 2-2A and said signal was observed on the surface of the LRRTM2 bead in Figure 2-2B, it is revealed that antibody was delivered to the surface of the LRRTM2 bead by targeting the N terminus of SYT2 only when motor neuron presynapse was present.

Furthermore, the signal intensity was higher in the condition with an antibody concentration of 10µg/mL (Figure 3B) compared to the condition of 1 µg/mL (Figure 2-1B), indicating that the increase of antibody amount at the presynapse is proportional to the concentration of the antibody added.

These results showed that an antibody against the intravesicular domain of Synaptotagmin 2 was delivered to the presynapse of motor neurons, and the amount thereof was dependent on antibody concentration.

### < Example 2: Delivery of Synaptotagmin 2 Antibody to Human Motor Neuron Synapse by Stimulation of Cells >

Whether an Synaptotagmin 2 antibody is delivered to a human motor neuron synapse by stimulating the neuron was examined.

Culture of cells, preparation of LRRTM2 beads, and induction of presynapse were performed in the same manner as in Example 1.

An antibody solution containing 4-aminopyridine was used to stimulate neurons. Antibody solution was prepared by the following method.

After warming the neuronal medium at 37°C for 30 minutes, 4-aminopyridine (Sigma Aldrich) was added to the medium to have a final concentration of 100µM and mixed. In addition, an antibody was added to have a final concentration of 2µg/mL and mixed. The crude antibody solution was centrifuged at 200g for 3 minutes at room temperature, and the supernatant was collected as an antibody solution.

Antibody and 4-aminopyridine were introduced by changing the culture medium with 100µL of antibody solution on the plate on which synapse was formed.

After introduction of the antibody and 4-aminopyridine, motor neurons were stimulated by culturing at room temperature for 10 minutes or 30 minutes.

Subsequent fixation of cells and immunocytochemical staining were performed as in Example 1.

Experiments on temperature conditions were performed in the same manner as described above except for temperature conditions.

As for the condition at the temperature of 37°C, motor neurons were stimulated by culturing at 37°C for 30 minutes after introducing the antibody and 4-aminopyridine.

As for the condition at the temperature of 4°C, motor neurons were stimulated by culturing at 4°C for 30 minutes after introducing the antibody and 4-aminopyridine.

For some motor neurons, motor neurons were stimulated by culturing at 4°C for 30 minutes, followed by culturing at an elevated temperature of 37°C for 30 minutes, after introducing the antibody and 4-aminopyridine.

The results are shown in Figures 4 and 5. Figures 4 and 5 show immunocytochemical staining images when the reaction times were 10 minutes and 30 minutes, respectively.

In control, in which a normal rabbit IgG antibody was used as an antibody, no signal was observed under conditions with any time period (Figure 4A and Figure 5A). On the other hand, where an anti-SYT2 N-terminal antibody was used as an antibody, a signal was observed on LRRTM2 beads under conditions with any time period (Figure 4B and Figure 5B). This suggests that anti-SYT2 N-terminal antibody is delivered to the presynapse not only by spontaneous activity of motor neurons but also by stimulating motor neurons with chemicals.

In addition, the signal intensity was slightly higher with a reaction time of 30 minutes (Figure 5B) compared to 10 minutes (Figure 4B), indicating that antibody amount at presynapse increased as the duration of the stimulus was increased. Similar experiments were also performed under a low-temperature condition (4°C), in which cellular activity is remarkably arrested, and the signal of the anti-SYT2 N-terminal antibody on LRRTM2 beads was remarkably reduced compared to the experiment at 37°C. Furthermore, the reduced signal was rescued by resetting the temperature from 4°C to 37°C.

These results showed that an antibody against the intravesicular domain of Synaptotagmin 2 was delivered to presynapse of motor neurons in a neuronal activity-dependent manner, and the amount thereof was dependent on the reaction time.

### < Example 3: Delivery of a Synaptotagmin 2 Antibody to Mouse Motor Neuron Synapses by Intravenous Injection >

Whether a Synaptotagmin 2 antibody introduced into the living body by intravenous injection was delivered to motor neuron synapses was examined.

The same antibody as Example 1 was used as an antibody to be introduced.

As an antibody solution, the mixture prepared by adding an antibody to PBS to have a final concentration of 1mg/mL and being mixed was used.

The antibody solution was administered to wild-type mice via tail vein injection at a dose of 5mg/kg.

Mice were sacrificed 12 hours, 24 hours, or 72 hours after administration, and gastrocnemius muscles were harvested and fixed.

The fixed gastrocnemius muscles were frozen together with tissue embedding agents, and sections of 10µm in thickness were prepared from the frozen tissue blocks using a cryostat.

Immunohistochemical staining was performed using the administered antibody as the primary antibody. For the tissue sections prepared, after blocking, primary antibody reaction was performed using α-bungarotoxin antibody (α-BgtX antibody Alexa Fluor 594 conjugate; Cat. No. B13423; Thermo Fisher Scientific) and Synapsin 1 antibody (SYN1 antibody; Cat. No. 106 308; Synaptic Systems) as additional primary antibodies. A secondary antibody reaction against each primary antibody was then performed to obtain fluorescent images. Blocking was performed using blocking buffer (PBS + 2% normal goat serum + 1% BSA + 1% fetal bovine serum + 0.02% TritonX-100).

The following antibodies were used as the secondary antibodies:
Alexa 488 labeled anti-rabbit antibody (Cat. No. A32731; Thermo Fisher Scientific);
Alexa 647 labeled anti-guinea pig antibody (Cat. No. A21450; Thermo Fisher Scientific).

Fluorescent images were obtained basically in the same manner as in Example 1. For detection of the signal of Alexa 647, the maximum excitation wavelength 650nm, the maximum detection wavelength 665nm, and the actual detection wavelength 705nm were used. For detection of the signal of Alexa 594, the maximum excitation wavelength 590nm, the maximum detection wavelength 617nm, and the actual detection wavelength 595nm were used. The following exposure times and detection thresholds were used for detection of each signal. All gamma correction values were 1.

Exposure Time and Detection Threshold:
Alexa 488 exposure time 150ms; detection threshold 130-1500;
Alexa 594 exposure time 100ms; detection threshold 100-2500;
Alexa 647 exposure time 100ms; detection threshold 130-1000.

The results are shown in Figures 6 and 7. Figures 6 and 7 show immunohistochemical staining images at 12 hours and 72 hours after administration, respectively. In this context, α-BgtX is a marker of acetylcholine receptors on the muscle cell membrane, and SYN1 is a marker of presynapse.

In control, in which a normal rabbit IgG antibody was used as an antibody, no signal of the normal rabbit IgG antibody was observed at the neuromuscular junctions, where α-BgtX and SYN1 signals were observed, under conditions with any time period (Figure 6A and Figure 7A).

On the other hand, where an anti-SYT2 N-terminal antibody was used as an antibody, the signal of the anti-SYT2 N-terminal antibody was observed at the neuromuscular junction under conditions with any time period (Figure 6B and Figure 7B).

Synaptotagmin 2 is known to be expressed on the presynapse of motor neuron synapses. This indicated that the anti-SYT2 N-terminal antibody administered by intravenous injection is delivered to the presynapse of motor neurons in vivo. In addition, since sufficient time has elapsed for endocytosis of synaptic vesicles, it is considered that the administered anti-SYT2 N-terminal antibody is incorporated into motor neuron synaptic vesicles.

In addition, the signal intensity was slightly higher at 72 hours (Figure 7B) compared to 12 hours (Figure 6B) after the administration of antibody, indicating that the antibody amount at the presynapse increased as the duration of antibody exposure increased.

In addition, no anti-SYT2 N-terminal antibody signal was detected in the liver, where the autonomic nerve projects, at 72 hours after administration of the antibody, and no anomaly was observed in the mouse individual or the liver.

Anti-SYT2 N-terminal antibody used was a complete IgG antibody and therefore does not cross the blood-brain barrier. It is also known that Synaptotagmin 2 is not expressed in sensory neurons. In addition, since no signal was observed in the liver, it was demonstrated that the antibody against the intravesicular domain of Synaptotagmin 2 was delivered to the presynapse of motor neurons by intravenous injection, as well as that the administration of the antibody exhibited no significant side effects, and that the antibody amount was shown to be dependent on the duration of the antibody exposure.

### < Example 4: Delivery of a Synaptotagmin 2 Antibody to Motor Neuron Synapses by Intraperitoneal Injection >

Whether a Synaptotagmin 2 antibody introduced into the living body by intraperitoneal injection was delivered to motor neuron synapses was examined.

Experiments were performed in the same manner as in Example 3, except that the administration was performed by intraperitoneal injection at a dose of 10 mg/kg.

The results are shown in Figures 8 and 9. Figures 8 and 9 show immunohistochemical staining images at 12 hours and 72 hours after administration, respectively. In this context, α-BgtX is a marker of acetylcholine receptors on the muscle cell membrane, and SYN1 is a marker of presynapse.

In control, in which a normal rabbit IgG antibody was used as an antibody, no signal of the normal rabbit IgG antibody was observed at the neuromuscular junctions, where α-BgtX and SYN1 signals were observed, under conditions with any time period (Figure 8A and Figure 9A).

On the other hand, where an anti-SYT2 N-terminal antibody was used as an antibody, the signal of the anti-SYT2 N-terminal antibody was observed at the neuromuscular junction under conditions with any time period (Figure 8B and Figure 9B). In addition, no anomalies were observed in the mouse individual and the liver, where the autonomic nerve projects, after administration of the antibody. This indicated that the anti-SYT2 N-terminal antibody was delivered to the presynapse of motor neurons by intraperitoneal injection, as was the case when the antibody was administered by tail vein injection.

### < Example 5: Delivery of a Synaptotagmin 2 Antibody to Mouse Motor Neuron Synaptic Vesicle by Intravenous Injection >

Whether a Synaptotagmin 2 antibody introduced into the living body by intravenous injection was delivered to synaptic vesicles of motor neurons was examined.

The same antibody as Example 1 was used as an antibody to be introduced.

As an antibody solution, the mixture prepared by adding an antibody to PBS to have a final concentration of 1mg/mL and being mixed was used.

The antibody solution was administered to wild-type mice via tail vein injection at a dose of 5mg/kg.

Mice were sacrificed 72 hours after administration, and the gastrocnemius muscles were harvested and fixed.

The fixed gastrocnemius muscles were frozen together with tissue embedding agents, and sections of 10µm in thickness were prepared from the frozen tissue blocks. After blocking, the sections were reacted with nanogold-labeled anti-rabbit antibody (Cat. No. A-24922; Thermo Fisher Scientific).

Sections were washed and then fixed with 2.5% glutaraldehyde, and the nanogold signal was enhanced using the R-gent Se-EM kit (Cat. No. 500.033; Aurion) and the HQ-silver kit (Cat. No. 2012; Nanoprobes). Sections were then fixed again with 1%OsO₄, dehydrated and embedded in Epon. Ultrathin sections (thickness 70nm) were produced from the embedded sections using an ultramicrotome (Cat. No. Leica EM UC7; Leica Microsystems). Sections were stained with uranium acetate and lead citrate and observed using transmission electron microscopy (Cat. No. JEM-1400plus, JEOL).

The results are shown in Figures 10 and 11. Figures 10 and 11 show transmission electron-microscopic images of the axon terminal of a motor neuron (the area surrounded by the solid white line in the figure) and gastrocnemius muscle cells (the area surrounded by the dashed black line in the figure). Black dots in the diagram represent the signal (silver-amplified signal) indicating the position of the nanogold of the antibody.

In control, where a normal rabbit IgG antibody is used as the antibody, only sparse antibody signals were observed in the neuronal axon terminal of the motor neuron, as well as in gastrocnemius muscle cells and other regions (Figure 10A). The signals observed here are considered to be background signals containing artifacts.

On the other hand, where an anti-SYT2 N-terminal antibody was used as the antibody, the antibody signals were remarkably observed in the neuronal axon terminal of the motor neuron compared to gastrocnemius muscle cells and other regions (Figure 10B). This indicates that the antibody was incorporated into the neuronal axon terminal of the motor neuron when an anti-SYT2 N-terminal antibody was used.

However, since the intracellular membrane structure observed at this magnification was predominantly mitochondria (arrowhead) and Junctional fold (pleated structure) of gastrocnemius muscle cells, it was not determined from this image whether the antibody was incorporated into synaptic vesicles.

Therefore, the samples obtained where an anti-SYT2 N-terminal antibody was used as the antibody were further magnified and observed (Figure 11). As a result, an antibody (b in Figure 11B) was observed to be incorporated into synaptic vesicle (a and b in Figure 11B) in the neuronal axon terminal of the motor neuron. This indicates that an anti-SYT2 N-terminal antibody is delivered into synaptic vesicles in the presynapse, which is located inside the cell, of the motor neuron, even when the antibody is administered to the living body.

### < Example 6: Delivery of a Synaptotagmin 2 Antibody to Mouse Motor Neurons by Intravenous Injection >

Whether a Synaptotagmin 2 antibody introduced into the living body by intravenous injection is delivered into motor neurons was examined.

The same antibody as Example 1 was used as an antibody to be introduced.

As an antibody solution, the mixture prepared by adding an antibody to PBS to have a final concentration of 1mg/mL and being mixed was used.

The antibody solution was administered to wild-type mice via tail vein injection at a dose of 5mg/kg.

Mice were sacrificed 72 hours after administration, and the spinal cord was harvested and fixed.

The fixed spinal cord was frozen together with tissue embedding agents, and sections of 10µm in thickness were prepared from the frozen tissue blocks using a cryostat.

Immunohistochemical staining was performed using the administered antibody as the primary antibody. For the tissue sections prepared, after blocking, a primary antibody reaction was performed using an anti-choline acetyltransferase antibody (α-ChAT antibody; Cat. No. #NBP1-30052; Novus BioLogicals) as an additional primary antibody. A secondary antibody reaction against each primary antibody was then performed to obtain fluorescent images. Blocking was performed using blocking buffer (PBS + 2% normal donkey serum + 1% BSA + 1% fetal bovine serum + 0.02% TritonX-100).

The same secondary antibody as Example 3 was used, and the fluorescent images were obtained in the same manner as in Example 3.

The results are shown in Figures 12-14. Figure 12 shows the immunohistochemical staining image when observed using an objective with 10× magnification (Figure 12) or 40× magnification (Figures 13 and 14). In this context, the α-ChAT is a marker of choline acetyltransferase in motor neuron.

As the anterior horn of the spinal cord (left side of the dashed line in Figure 12) was observed using a 10× objective, the signal of the anti-SYT2 N-terminal antibody was observed in most cell bodies of motor neurons where the signal of α-ChAT was present (Figure 12: arrowhead).

Observation using a further higher magnification of 40× objective showed that in control, where a normal rabbit IgG antibody was used as the antibody, no signal of the normal rabbit IgG antibody was observed in the cell body of motor neurons where a signal of α-ChAT was present (Figure 13).

On the other hand, where an anti-SYT2 N-terminal antibody was used as the antibody, a signal of the anti-SYT2 N-terminal antibody was also observed in the cell bodies of motor neurons (Figure 14: arrowhead).

This suggested that intravenously administered anti-SYT2 N-terminal antibody is incorporated into synaptic vesicle at synapse located at the neuromuscular junction and then retrogradely transported through the axon and delivered to the cell body.

### < Example 7: Observation of the Synaptotagmin 2 Antibody Delivered by Intravenous Injection Over Time >

The signal of the Synaptotagmin 2 antibody introduced into the living body by intravenous injection and delivered into motor neurons was observed over time.

The same antibody as Example 1 was used as an antibody to be introduced.

As an antibody solution, the mixture prepared by adding an antibody to PBS to have a final concentration of 1mg/mL and being mixed was used.

Spinal cord collected was administered to wild-type mice by tail vein injection at a dose of 5mg/kg.

Sampling of the spinal cord was performed as in Example 7, except that it was performed at 6 hours, 24 hours, 72 hours, 120 hours, 168 hours, and 240 hours after administration.

The results are shown in Figure 15 and 16. Figure 15 shows immunohistochemical staining images when harvested 6-72 hours after administration, and Figure 16 shows immunohistochemical staining image when harvested 120-240 hours after administration.

The anti-SYT2 N-terminal antibody signal was also observed in the sample at 6 hours after administration (Figure 15), and the anti-SYT2 N-terminal antibody signal was observed in the samples at any time point thereafter up to 240 hours after administration (Figures 15 and 16).

In addition, there was a general tendency that signals become stronger over time. Accordingly, it is estimated that the anti-SYT2 N-terminal antibody is delivered to the cell body by 6 hours after its administration, and then accumulates over time.

### < Example 8: Varification of Pharmacological Effect by Delivery of Drugs Using Synaptotagmin 2 Antibody >

Using a Synaptotagmin 2 antibody that was confirmed to be delivered into the motor neuron, it was examined whether drug-based pharmacological effect was observed when a drug was delivered into the motor neuron.

### 1. Induction of the Motor Neuron Presynapse

Experiments were performed using human motor neurons, for which synaptogenesis was induced using microbeads coated with the extracellular domain of LRRTM2. Culture of cells, preparation of LRRTM2 beads and induction of presynapse were performed as described in Example 1.

### 2. Preparation of a Conjugate

As the drug, a microtubule polymerization inhibitor monomethylauristatin E (MMAE: Cat. No. #HY-15575; MedChemExpress) was used. As the anti-SYT2 N-terminal antibody and the normal rabbit antibody for control, antibodies used in Example 1 were used. Conjugate of MMAE and the antibody was prepared using MagicLink^{™} kit (broadpharm). Preparation of conjugate was performed according to the manufacturer's protocols.

### 3. Preparation of Conjugate Solutions

After warming the neuronal medium at 37°C for 30 minutes, 4-aminopyridine (Sigma Aldrich) was added to the medium to have a final concentration of 100µM and mixed. In addition, conjugate was added to have a final concentration of 1µg/mL and mixed. The crude conjugate solution was centrifuged at 200g for 3 minutes at room temperature and the supernatant was collected as a conjugate solution.

As a further control group, MMAE alone instead of conjugate was introduced for some samples (sample size: 13). For this group, MMAE solution was prepared as described above using MMAE instead of conjugate at the same concentration.

### 4. Introduction of the Conjugate

The conjugate was introduced in a plate, on which presynapse was formed, by changing media with 100µL of conjugate solution or MMAE solution and culturing at 37°C for 30 minutes.

### 5. Axonal Elongation Reaction

After introduction, the solutions were collected and washed, and the medium was changed with neuronal medium without conjugate and MMAE. The axons were then elongated by culturing for an additional 24 hours.

### 6. Fixation, Staining and Observation of Cells

Cells were fixed in the same manner as in Example 1, and a primary antibody reaction using mouse anti-βIII tubulin (Tuj1) antibody and a secondary antibody reaction using Alexa 555 labeled anti-mouse antibody were performed in the same manner as in Example 1. In this Example, staining for the anti-SYT2 N-terminal antibody was not performed. Fluorescent images were obtained as Example 1. The sample sizes were as follows: the MMAE simple introduction group, 13 samples; the control antibody group, 17 samples; the SYT2 antibody group, 27 samples.

### 7. Analysis of Axonal Amount

The axonal amount was obtained as the sum of the brightness (= (fluorescence intensity per pixel) × (pixel number)) of Tuj1 signals in the obtained fluorescence images. The relative amount of axon was calculated as the value normalized by the results obtained using the conjugate of the control normal rabbit antibody and MMAE set to 100%. Brightness was obtained using LAS X software (Leica).

### 8. Statistical Analysis

Statistical analysis was performed by t-test using GraphPad Prism9 (GraphPad Software). The significance level used was p<0.05.

The results are shown in Figures 17-19. Figures 17 and 18 are immunocytochemical staining images showing the images of axons of the control antibody group (Figure 17), in which the conjugate of the control normal rabbit antibody and MMAE is used, and the SYT2 antibody group (Figure 18), in which the conjugate with anti-SYT2 N-terminal antibody is used. In addition, Figure 19 is a graph showing the results quantitatively.

In all experimental conditions, axons were elongated from the neurospheres, and the presynapse was normally induced (Figures 17A and 18A).

MMAE inhibits the polymerization of microtubules, the cytoskeleton that is critical for axon elongation and maintenance. Therefore, it is expected that the stronger the effectiveness of MMAE, the more inhibited the elongation and maintenance of axons and result in a decrease in the axonal amount.

In the control antibody group, many thick axons and the presynapse, which are observed as bulges, were observed even in the distal portion distal to the neurosphere (Figure 17B). In addition, in the proximal portion relatively close to the neurosphere, the axons were observed to run in an aligned manner in which the axons are almost parallel to each other (Figure 17C).

On the other hand, in the SYT2 antibody group, only a small number of thin axons were observed in the distal portion distal to the neurosphere, and presynapse was poorly formed (Figure 18B). In addition, in the proximal portion relatively close to the neurosphere, the axon was not aligned as in the control antibody group, and it was observed that the axon was disordered (Figure 18C).

The quantitative results also supported these observations. Compared with the control antibody group ("Cont. IgG-MMAE" in Figure 19), the axonal amount was drastically decreased, and the relative amount of axon was about 87.15% in the SYT2 antibody group ("α-SYT2 IgG-MMAE" in Figure 19). Additionally, the results of the SYT2 antibody group were significantly decreased even compared with the simple introduction group ("MMAE" in Figure 19), in which MMAE was introduced alone, and the axonal amount was about 82.7% of that of the simple introduction group. There were no significant differences between the simple introduction group and the control antibody group.

### < Example 9: Confirmation of the Relationship Between Pharmacological Effects and Neuronal Activity >

By inhibiting the uptake function of synaptic vesicle in neurons, it was confirmed that the pharmacological effects observed in Example 9 using the anti-SYT2 N-terminal antibody were dependent on the uptake of synaptic vesicle by neuronal activity.

Experiments were conducted only using the conjugate of the anti-SYT2 N-terminal antibody and MMAE. As a group for inhibiting the uptake of synaptic vesicle in neurons, an experimental group in which synaptogenesis was inhibited (synapse non-forming group) and an experimental group in which endocytosis was inhibited (the endocytosis-inhibiting group) were used. Experiments were performed in the same manner as in Example 9. except that the following process was performed on motor neurons in the synapse non-forming group and the endocytosis-inhibiting group.

**In** the synapse non-forming group, the experiments were performed using microbeads that were not coated with the extracellular domain of LRRTM2 as the beads to be used to induce the presynapse.

**In** the endocytosis-inhibiting group, the culture upon introducing the conjugate was performed under the condition of at 4°C for 30 minutes.

The sample sizes were 3 samples per group.

**In** this Example, the relative amount of axon normalized by the axonal amount in the synapse non-forming group set to 100% was used.

The results are shown in Figures 20-23. Figures 20-22 are immunocytochemical staining images showing images of axons of the normal firing group (Figure 20), in which the experiments were performed under the same conditions as Example 9, the synapse non-forming group (Figure 21), in which the presynapse was not induced, and the endocytosis-inhibiting group (Figure 22), in which endocytosis was inhibited by the low-temperature treatment. In addition, Figure 23 is a graph showing the results quantitatively.

**In** all experimental conditions, axons were elongated from the neurospheres (Figures 20-22).

In the normal firing group, similar to the results shown in Figure 18, the number of distal axons was small, and the elongation and maintenance of axons were inhibited by the effects of MMAE (Figure 20). In contrast, in both the synapse non-forming group (Figure 21) and the endocytosis-inhibiting group (Figure 22), the axon was elongated to the distal portion, and the effects of MMAE were observed to be limited.

The quantitative results also supported these observations (Figure 23). In the normal firing group, the amount of axon was significantly decreased compared to the synapse non-forming group, and the relative amount of axon was 81.8%. Additionally, the results of the normal firing group were significantly decreased even compared to the endocytosis inhibition group, and the axonal amount was about 80.6% of that of the endocytosis-inhibiting group. There were no significant differences between the synapse non-forming group and the endocytosis-inhibiting group.

These results indicate that the inhibition of the uptake function of synaptic vesicle by, such as inhibition of synaptogenesis or inhibition of endocytosis, almost completely suppresses the pharmacological effects of the conjugate of the anti-SYT2 N-terminal antibody and MMAE. Therefore, it was suggested that the pharmacological effects of the conjugate of the anti-SYT2 N-terminal antibody and MMAE are mediated by neuronal activity, particularly endocytosis in synapse.

### < Example 10: Validation of Pharmacological Effect When Targeting Other Membrane Proteins >

Antibodies to other membrane proteins present in synaptic vesicles were also examined to determine whether they could be utilized in drug delivery, as with the anti-SYT2 N-terminal antibody.

Experiments were performed in the same manner as in Example 9, except for the antibody used. Antibodies used were as follows:
Anti-SYT2 N-terminal antibody: Polyclonal rabbit purified antibody SYT2 lumenal domain (Cat. No. 105 223; Synaptic Systems); sample size: 27;
Anti-SYP antibody: Anti-Synaptophysin Antibody Major synaptic vesicle protein p38, SYP (Cat. No. ANR-013; alomone labs; Epitope: SEQ ID NO: 21); sample size: 20;
Anti-SYNGR1 antibody: Synaptogyrin 1 Antibody-BSA Free (Cat. No. NBP1-77371; Novus Biologicals; epitope: SEQ ID NO: 17); sample size: 20;
Anti-SYT1 antibody: Synaptotagmin1 antibody luminal domain (Cat. No. 105 103; Synaptic Systems; epitope: SEQ ID NO: 24); sample size: 15;
Anti-SV2A antibody: SV2A Polyclonal Antibody (Cat. No. BS-2407R; Bioss antibodies; epitope: SEQ ID NO: 13); sample size: 14.

In this Example, the relative amount of axon was calculated as the value normalized by the results obtained using the conjugate of the control normal rabbit antibody and MMAE set to 100%.

The results are shown in Figure 24. Figure 24 shows the relative amount of axon when the conjugate, in which each antibody was used, was introduced.

Even when the membrane protein present on synaptic vesicle other than the anti-SYT2 N-terminal antibody was used, the axonal amount was significantly decreased, and the relative amount of axon was 87.2% to 90.7% (Figure 24). Depending on the type of antibody used, there were no significant differences in the relative amount of axon.

Therefore, it was confirmed that the drug or the like can be delivered to the motor neuron by using antibodies against membrane proteins present on synaptic vesicles, regardless of the type of membrane protein to be targeted. In addition, it was suggested that with such targeting, effects based on the drug or the like can be imparted to the target cell.

All publications, patents and patent applications cited in the description are incorporated in their entirety by reference.

## Claims

1. A targeting agent to a motor neuron comprising an antibody capable of binding to the intravesicular domain of a membrane protein present on the synaptic vesicle of a motor neuron.

2. The targeting agent according to claim 1, further comprising a label substance and/or a physiologically active substance.

3. The targeting agent according to claim 2, comprising a conjugate of the antibody and the label substance and/or physiologically active substance.

4. The targeting agent according to claim 1, wherein the membrane protein is a human-derived protein.

5. The targeting agent according to claim 1, wherein the membrane protein comprises any one protein selected from the group consisting of Synaptotagmin 2, Synaptic vesicle glycoprotein 2A, Synaptogyrin 1, Synaptophysin, and Synaptotagmin 1.

6. The targeting agent according to claim 1, wherein the intravesicular domain is the domain comprising 4 or more amino acids.

7. The targeting agent according to claim 5, wherein the intravesicular domain consists of the amino acid sequence of any one selected from the group consisting of SEQ ID NOs: 3, 7-12, 15, 16, 19, 20, and 23; the amino acid sequence having addition, deletion, and/or substitution of one or multiple amino acid in amino acid sequence of any one selected from the group consisting of SEQ ID NOs: 3, 7-12, 15, 16, 19, 20, and 23; the amino acid sequence having the sequence identity of 90% or more to the amino acid sequence of any one selected from the group consisting of SEQ ID NOs: 3, 7-12, 15, 16, 19, 20, and 23.

8. The targeting agent according to claim 2, wherein the label substance is a fluorescent molecule.

9. The targeting agent according to claim 2 comprising the label substance, wherein the targeting agent is a motor neuron visualizing agent.

10. The targeting agent according to claim 2, wherein the physiologically active substance is one or more selected from the group consisting of a synaptogenesis-promoting agent, a synapse-maintaining agent, a muscle-enhancing agent, and a neuronal function-modifying agent.

11. The targeting agent according to claim 1, wherein the targeting agent is incorporated intracellularly by endocytosis.

12. A pharmaceutical composition comprising the targeting agent according to claim 2.

13. A method for targeting the label substance and/or physiologically active substance to a motor neuron comprising a step of contacting the targeting agent according to claim 2 and/or the pharmaceutical composition according to claim 12 with a motor neuron; and a step of delivering the targeting agent and/or pharmaceutical composition to a synapse of the motor neuron.

14. A method for visualizing a motor neuron comprising a step of contacting the motor neuron visualizing agent according to claim 9 with a motor neuron; a step of delivering the motor neuron visualizing agent to a synapse of the motor neuron; and a step of detecting a signal of the label substance.
